# EUROPÄISCHE PATENTSCHRIFT

(11) **EP 0 667 909 B1**
(45) Veröffentlichungstag und Bekanntmachung des Hinweises auf die Patenterteilung: **11.02.1998**
(21) Anmeldenummer: 93920855.9
(22) Anmeldetag: 01.10.1993
(51) Int. Cl.: C12N 15/67, C12N 15/52, C12N 15/70, C12N 1/21, C12P 17/18

(54) **BIOTECHNOLOGISCHES VERFAHREN ZUR HERSTELLUNG VON BIOTIN**
BIOTECHNOLOGICAL METHOD OF PRODUCING BIOTIN
PROCEDE BIOTECHNOLOGIQUE DE PREPARATION DE BIOTINE

(30) Priorität: 02.10.1992 CH 3124/92; 15.07.1993 CH 2134/93
(43) Veröffentlichungstag der Anmeldung: 23.08.1995
(60) Teilanmeldung: 97107803.5
(73) Patentinhaber: LONZA AG, CH-3945 Gampel/Wallis (CH)
(72) Erfinder: BIRCH, Olwen, CH-3904 Naters (CH); BRASS, Johann, CH-3938 Ausserberg (CH); FUHRMANN, Martin, CH-3930 Visp (CH); SHAW, Nicholas, CH-3930 Visp (CH)
(74) Vertreter: Weinhold, Peter, Dr.
(86) Internationale Anmeldenummer: EP9302688
(87) Internationale Veröffentlichungsnummer: WO9408023

(56) Entgegenhaltungen:
- EP-A- 0 266 240
- EP-A- 0 316 229
- EP-A- 0 449 724
- WO-A-87/01391
- GB-A- 2 216 530
- AGRIC. BIOL. CHEM., Bd. 50, Nr. 2, 1986, TOKYO, JAPAN, Seiten 499-500; Y. KAWAGUCHI ET AL.: 'Improved direct expression of Prochymosin cDNA through changing the SD-ATG codon length'
- DNA, Bd. 2, Nr. 3, 1983, MARY ANN LIEBERT, INC. PUBLISHERS, Seiten 231-235; H.A. DE BOER ET AL.: 'Portable shine-dalgarno regions: A system study of defined alterations of the nucleotide sequences within E. coli ribosome binding sites'
- JOURNAL OF BIOTECHNOLOGY, Bd. 9, 1989, ELSEVIER, AMSTERDAM, THE NETHERLANDS, Seiten 179-190; M.K.OLSEN ET AL.: 'Enhancement of heterologous polypeptide expression by alterations in the ribosome-binding-site sequence'
- GENE, Bd. 67, Nr. 2, 1988, ELSEVIER PUBLISHERS, N.Y., U.S., Seiten 203-211; D. SHIUAN AND A. CAMPBELL: 'Transcriptional regulation and gene arrangement of Escherichia coli, Citrobacter freundii and Salmonella typhimurium biotin operons'
- BIOSCI. BIOTECH. BIOCHEM., Bd. 56, Nr. 11, November 1992, TOKYO,JP, Seiten 1780-1785; O. IFUKU ET AL.: 'Conversion of dethiobiotin to biotin in cell-free extracts of Escherichia coli'
- FEMS MICROBIOLGY LETTERS, Bd. 110, Nr. 1, 1. Juni 1993, ELSEVIER, AMSTERDAM, NL, Seiten 1-4; A. FUJISAWA ET AL.: 'Bioconversion of dethiobiotin to biotin by a cell-free system of a bioYB transformant of Bacillus shaericus'

### SEQUENZPROTOKOLL

(1) ALGEMEINE INFORMATION:
   (i) ANMELDER:
      (A) NAME: LONZA AG
      (B) STRASSE: Muenchensteinerstrasse 38
      (C) ORT: Basel
      (E) LAND: Schweiz
      (F) POSTLEITZAHL: 4002
   (ii) ANMELDETITEL: Biotechnologisches Verfahren zur Herstellung von Biotin
   (iii) ANZAHL DER SEQUENZEN: 19
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
   (vi) FRÜHERE ANMELDEDATEN:
      (A) ANMELDENUMMER: CH 3124/92
      (B) ANMELDEDATUM: 02-OCT-1992
   (vi) FRÜHERE ANMELDEDATEN:
      (A) ANMELDENUMMER: CH 2134/93
      (B) ANMELDEDATUM: 15-JUL-1993
(2) INFORMATION ZU SEQ ID NO: 1:
   (i) SEQUENZ CHARAKTERISTIKA:
      (A) LÄNGE: 5872 Basenpaare
      (B) ART: Nukleinsäure
      (C) STRANGFORM: Doppel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (A) ORGANISMUS: Escherichia coli
      (B) STAMM: DSM498
   (vii) UNMITTELBARE HERKUNFT:
      (B) CLON: pBO30A-15/9
   (ix) MERKMALE:
      (A) NAME/SCHLÜSSEL: CDS
      (B) LAGE: 117..1157
      (C) ART DER ERMITTLUNG: experimentell
      (D) SONSTIGE ANGABEN: /codon_start= 117
         /product= "Biotin Synthase"
         /evidence= EXPERIMENTAL
         /gene= "bioB"
         /number= 1
   (ix) MERKMALE:
      (A) NAME/SCHLÜSSEL: CDS
      (B) LAGE: 2295..3050
      (D) SONSTIGE ANGABEN: /codon_start= 2295
         /function= "involved in pimeloyl-CoA synthesis"
         /product= "protein"
         /gene= "bioC"
         /number= 3
   (ix) MERKMALE:
      (A) NAME/SCHLÜSSEL: CDS
      (B) LAGE: 3750..5039
      (C) ART DER ERMITTLUNG: experimentell
      (D) SONSTIGE ANGABEN: /codon_start= 3750
         /EC_number= 2.6.1.62
         /procuct= "DAPA synthase"
         /evidence= EXPERIMENTAL
         /gene= "bioA"
         /number= 5
         /standard_name=
         "S-Adenosyl-L-methionine:8-amino-7-oxononanoate
         aminotransf."
   (ix) MERKMALE:
      (A) NAME/SCHLÜSSEL: CDS
      (B) LAGE: 5098.. 5574
      (C) ART DER ERMITTLUNG: experimentell
      (D) SONSTIGE ANGABEN: /codon_start= 5098
         /function= "unknown, involved in biotin synthesis"
         /product= "protein"
         /evidence= EXPERIMENTAL
         /gene= "ORFI"
         /number= 6
   (ix) MERKMALE:
      (A) NAME/SCHLÜSSEL: -10_signal
      (B) LAGE: 45..49
      (C) ART DER ERMITTLUNG: experimentell
      (D) SONSTIGE ANGABEN: /evidence= EXPERIMENTAL
         /standard_name = "promoter ptac"
   (ix) MERKMALE:
      (A) NAME/SCHLÜSSEL: -35_signal
      (B) LAGE: 23..28
      (D) SONSTIGE ANGABEN: /standard_name= "promoter ptac"
   (ix) MERKMALE:
      (A) NAME/SCHLÜSSEL: RBS
      (B) LAGE: 105..119
      (C) ART DER ERMITTLUNG: experimentell
      (D) SONSTIGE ANGABEN: /evidence= EXPERIMENTAL
         /standard_name= "bioB RBS no.9"
   (ix) MERKMALE:
      (A) NAME/SCHLÜSSEL: RBS
      (B) LAGE: 2284..2297
      (D) SONSTIGE ANGABEN: /standard_name= "bioC RBS"
   (ix) MERKMALE:
      (A) NAME/SCHLÜSSEL: RBS
      (B) LAGE: 3742..3752
      (D) SONSTIGE ANGABEN: /standard_name= "bioA RBS"
   (ix) MERKMALE:
      (A) NAME/SCHLÜSSEL: RBS
      (B) LAGE: 5088. .5100
      (D) SONSTIGE ANGABEN: /standard_name= "ORFI RBS"
   (ix) MERKMALE:
      (A) NAME/SCHLÜSSEL: terminator
      (B) LAGE: 5583..5644
      (D) SONSTIGE ANGABEN: /standard_name= "rho-independent
         transcriptional terminator"
   (ix) MERKMALE:
      (A) NAME/SCHLÜSSEL: stem_ loop
      (B) LAGE: 5583..5605
   (ix) MERKMALE:
      (A) NAME/SCHLÜSSEL: promoter
      (B) LAGE: 1..96
      (C) ART DER ERMITTLUNG: experimentell
      (D) SONSTIGE ANGABEN: /function= "promoter ptac"
         /evidence= EXPERIMENTAL
   (x) VERÖFFENTLICHUNGSINFORMATION:
      (H) DOCUMENTNUMMER: WO 87/01391 B1
      (I) ANMELDEDATUM: 26-AUG-1986
      (J) VERÖFFENTLICHUNGSTAG: 07-APR-1993
   (xi) SEQUENZBESCHREIBUNG: SEQ ID NO: 1:
   AAGCTTACTC CCCATCCCCC TGTTGACAAT TAATCATCGG CTCGTATAAT GTGTGGAATT      60
   GTGAGCGGAT AACAATTTCA CACAGGAAAC AGGATCGGTA CCTAAGGAGA CTAGTC         116
   ATG GCT CAC CGC CCA CGC TGG ACA TTG TCG CAA GTC ACA GAA TTA TTT       164
   Met Ala His Arg Pro Arg Trp Thr Leu Ser Gln Val Thr Glu Leu Phe
     1               5                  10                  15
   GAA AAA CCG TTG CTG GAT CTG CTG TTT GAA GCG CAG CAG GTG CAT CGC       212
   Glu Lys Pro Leu Leu Asp Leu Leu Phe Glu Ala Gln Gln Val His Arg
                20                  25                  30
   CAG CAT TTC GAT CCT CGT CAG GTG CAG GTC AGC ACG TTG CTG TCG ATT       260
   Gln His Phe Asp Pro Arg Gln Val Gln Val Ser Thr Leu Leu Ser Ile
            35                  40                  45
   AAG ACC GGA GCT TGT CCG GAA GAT TGC AAA TAC TGC CCG CAA AGC TCG       308
   Lys Thr Gly Ala Cys Pro Glu Asp Cys Lys Tyr Cys Pro Gln Ser Ser
        50                  55                  60
   CGC TAC AAA ACC GGG CTG GAA GCC GAG CGG TTG ATG GAA GTT GAA CAG       356
   Arg Tyr Lys Thr Gly Leu Glu Ala Glu Arg Leu Met Glu Val Glu Gln
    65                  70                  75                  80
   GTG CTG GAG TCG GCG CGC AAA GCG AAA GCG GCA GGA TCG ACG CGC TTC       404
   Val Leu Glu Ser Ala Arg Lys Ala Lys Ala Ala Gly Ser Thr Arg Phe
                85                  90                  95
   TGT ATG GGC GCG GCG TGG AAG AAT CCC CAC GAA CGC GAT ATG CCG TAC       452
   Cys Met Gly Ala Ala Trp Lys Asn Pro His Glu Arg Asp Met Pro Tyr
               100                 105                 110
   CTG GAA CAA ATG GTG CAG GGG GTA AAA GCG ATG GGG CTG GAG GCG TGT       500
   Leu Glu Gln Met Val Gln Gly Val Lys Ala Met Gly Leu Glu Ala Cys
           115                 120                 125
   ATG ACG CTG GGC ACG TTG AGT GAA TCT CAG GCG CAG CGC CTC GCG AAC       548
   Met Thr Leu Gly Thr Leu Ser Glu Ser Gln Ala Gln Arg Leu Ala Asn
   130 135 140
   GCC GGG CTG GAT TAC TAC AAC CAC AAC CTG GAC ACC TCG CCG GAG TTT 596
   Ala Gly Leu Asp Tyr Tyr Asn His Asn Leu Asp Thr Ser Pro Glu Phe
   145 150 155 160
   TAC GGC AAT ATC ATC ACC ACA CGC ACT TAT CAG GAA CGC CTC GAT ACG 644
   Tyr Gly Asn Ile Ile Thr Thr Arg Thr Tyr Gln Glu Arg Leu Asp Thr
   165 170 175
   CTG GAA AAA GTG CGC GAT GCC GGG ATC AAA GTC TGT TCT GGC GGC ATT 692
   Leu Glu Lys Val Arg Asp Ala Gly Ile Lys Val Cys Ser Gly Gly Ile
   180 185 190
   GTG GGC TTA GGC GAA ACG GTA AAA GAT CGC GCC GGA TTA TTG CTG CAA 740
   Val Gly Leu Gly Glu Thr Val Lys Asp Arg Ala Gly Leu Leu Leu Gln
   195 200 205
   CTG GCA AAC CTG CCG ACG CCG CCG GAA AGC GTG CCA ATC AAC ATG CTG 788
   Leu Ala Asn Leu Pro Thr Pro Pro Glu Ser Val Pro Ile Asn Met Leu
   210 215 220
   GTG AAG GTG AAA GGC ACG CCG CTT GCC GAT AAC GAT GAT GTC GAT GCC 836
   Val Lys Val Lys Gly Thr Pro Leu Ala Asp Asn Asp Asp Val Asp Ala
   225 230 235 240
   TTT GAT TTT ATT CGC ACC ATT GCG GTC GCG CGG ATC ATG ATG CCA ACC 884
   Phe Asp Phe Ile Arg Thr Ile Ala Val Ala Arg Ile Met Met Pro Thr
   245 250 255
   TCT TAC GTG CGC CTT TCT GCC GGA CGC GAG CAG ATG AAC GAA CAG ACT 932
   Ser Tyr Val Arg Leu Ser Ala Gly Arg Glu Gln Met Asn Glu Gln Thr
   260 265 270
   CAG GCG ATG TGC TTT ATG GCA GGC GCA AAC TCG ATT TTC TAC GGT TGC 980
   Gln Ala Met Cys Phe Met Ala Gly Ala Asn Ser Ile Phe Tyr Gly Cys
   275 280 285
   AAA CTG CTG ACC ACG CCG AAT CCG GAA GAA GAT AAA GAC CTG CAA CTG 1028
   Lys Leu Leu Thr Thr Pro Asn Pro Glu Glu Asp Lys Asp Leu Gln Leu
   290 295 300
   TTC CGC AAA CTG GGG CTA AAT CCG CAG CAA ACT GCC GTG CTG GCA GGG 1076
   Phe Arg Lys Leu Gly Leu Asn Pro Gln Gln Thr Ala Val Leu Ala Gly
   305 310 315 320
   GAT AAC GAA CAA CAG CAA CGT CTT GAA CAG GCG CTG ATG ACC CCG GAC 1124
   Asp Asn Glu Gln Gln Gln Arg Leu Glu Gln Ala Leu Met Thr Pro Asp
   325 330 335
   ACC GAC GAA TAT TAC AAC GCG GCA GCA TTA TGAGCTGGCA GGAGAAAATC 1174
   Thr Asp Glu Tyr Tyr Asn Ala Ala Ala Leu
   340 345
   AACGCGGCGC TCGATGCGCG GCGTGCTGCC GATGCCCTGC GTCGCCGTTA TCCGGTGGCG 1234
   CAAGGAGCCG GACGCTGGCT GGTGGCGGAT GATCGCCAGT ATCTGAACTT TTCCAGTAAC 1294
   GATTATTTAG GTTTAAGCCA TCATCCGCAA ATTATCCGTG CCTGGCAGCA GGGGGCGGAG 1354
   CAATTTGGCA TCGGTAGCGG CGGCTCCGGT CACGTCAGCG GTTATAGCGT GGTGCATCAG 1414
   GCACTGGAAG AAGAGCTGGC CGAGTGGCTT GGCTATTCGC GGGCACTGCT GTTTATCTCT 1474
   Met Thr Leu Gly Thr Leu Ser Glu Ser Gln Ala Gln Arg Leu Ala Asn
   130 135 140
   GGTTTCGCCG CTAATCAGGC AGTTATTGCC GCGATGATGG CGAAAGAGGA CCGTATTGCT   1534
   GCCGACCGGC TTAGCCATGC CTCATTGCTG GAAGCTGCCA GTTTAAGCCC GTCGCAGCTT   1594
   CGCCGTTTTG CTCATAACGA TGTCACTCAT TTGGCGCGAT TGCTTGCTTC CCCCTGTCCG   1654
   GGGCAGCAAA TGGTGGTGAC AGAAGGCGTG TTCAGCATGG ACGGCGATAG TGCGCCACTG   1714
   GCGGAAATCC AGCAGGTAAC GCAACAGCAC AATGGCTGGT TGATGGTCGA TGATGCCCAC   1774
   GGCACGGGCG TTATCGGGGA GCAGGGGCGC GGCAGCTGCT GGCTGCAAAA GGTAAAACCA   1834
   GAATTGCTGG TAGTGACTTT TGGCAAAGGA TTTGGCGTCA GCGGGGCAGC GGTGCTTTGC   1894
   TCCAGTACGG TGGCGGATTA TCTGCTGCAA TTCGCCCGCC ACCTTATCTA CAGCACCAGT   1954
   ATGCCGCCCG CTCAGGCGCA GGCATTACGT GCGTCGCTGG CGGTCATTCG CAGTGATGAG   2014
   GGTGATGCAC GGCGCGAAAA ACTGGCGGCA CTCATTACGC GTTTTCGTGC CGGAGTACAG   2074
   GATTTGCCGT TTACGCTTGC TGATTCATGC AGCGCCATCC AGCCATTGAT TGTCGGTGAT   2134
   AACAGCCGTG CGTTACAACT GGCAGAAAAA CTGCGTCAGC AAGGCTGCTG GGTCACGGCG   2194
   ATTCGCCCGC CAACCGTACC CGCTGGTACT GCGCGACTGC GCTTAACGCT AACCGCTGCG   2254
   CATGAAATGC AGGATATCGA CCGTCTGCTG GAGGTGCTGC ATG GCA ACG GTT AAT    2309
                                               Met Ala Thr Val Asn
                                                1               5
   AAA CAA GCC ATT GCA GCG GCA TTT GGT CGG GCA GCC GCA CAC TAT GAG    2357
   Lys Gln Ala Ile Ala Ala Ala Phe Gly Arg Ala Ala Ala His Tyr Glu
                    10                  15                  20
   CAA CAT GCA GAT CTA CAG CGC CAG AGT GCT GAC GCC TTA CTG GCA ATG    2405
   Gln His Ala Asp Leu Gln Arg Gln Ser Ala Asp Ala Leu Leu Ala Met
                25                  30                  35
   CTT CCA CAG CGT AAA TAC ACC CAC GTA CTG GAC GCG GGT TGT GGA CCT    2453
   Leu Pro Gln Arg Lys Tyr Thr His Val Leu Asp Ala Gly Cys Gly Pro
            40                  45                  50
   GGC TGG ATG AGC CGC CAC TGG CGG GAA CGT CAC GCG CAG GTG ACG GCC    2501
   Gly Trp Met Ser Arg His Trp Arg Glu Arg His Ala Gln Val Thr Ala
        55                  60                  65
   TTA GAT CTC TCG CCG CCA ATG CTT GTT CAG GCA CGC CAG AAG GAT GCC    2549
   Leu Asp Leu Ser Pro Pro Met Leu Val Gln Ala Arg Gln Lys Asp Ala
    70                  75                  80                  85
   GCA GAC CAT TAT CTG GCG GGA GAT ATC GAA TCC CTG CCG TTA GCG ACT    2597
   Ala Asp His Tyr Leu Ala Gly Asp Ile Glu Ser Leu Pro Leu Ala Thr
                    90                  95                 100
   GCG ACG TTC GAT CTT GCA TGG AGC AAT CTC GCA GTG CAG TGG TGC GGT    2645
   Ala Thr Phe Asp Leu Ala Trp Ser Asn Leu Ala Val Gln Trp Cys Gly
                105                 110                 115
   AAT TTA TCC ACG GCA CTC CGC GAG CTG TAT CGG GTG GTG CGC CCC AAA    2693
   Asn Leu Ser Thr Ala Leu Arg Glu Leu Tyr Arg Val Val Arg Pro Lys
            120                 125                 130
   GGC GTG GTC GCG TTT ACC ACG CTG GTG CAG GGA TCG TTA CCC GAA CTG    2741
   Gly Val Val Ala Phe Thr Thr Leu Val Gln Gly Ser Leu Pro Glu Leu
   135 140 145
   CAT CAG GCG TGG CAG GCG GTG GAC GAG CGT CCG CAT GCT AAT CGC TTT 2789
   His Gln Ala Trp Gln Ala Val Asp Glu Arg Pro His Ala Asn Arg Phe
   150 155 160 165
   TTA CCG CCA GAT GAA ATC GAA CAG TCG CTG AAC GGC GTG CAT TAT CAA 2837
   Leu Pro Pro Asp Glu Ile Glu Gln Ser Leu Asn Gly Val His Tyr Gln
   170 175 180
   CAT CAT ATT CAG CCC ATC ACG CTG TGG TTT GAT GAT GCG CTC AGT GCC 2885
   His His Ile Gln Pro Ile Thr Leu Trp Phe Asp Asp Ala Leu Ser Ala
   185 190 195
   ATG CGT TCG CTG AAA GGC ATC GGT GCC ACG CAT CTT CAT GAA GGG CGC 2933
   Met Arg Ser Leu Lys Gly Ile Gly Ala Thr His Leu His Glu Gly Arg
   200 205 210
   GAC CCG CGA ATA TTA ACG CGT TCG CAG TTG CAG CGA TTG CAA CTG GCC 2981
   Asp Pro Arg Ile Leu Thr Arg Ser Gln Leu Gln Arg Leu Gln Leu Ala
   215 220 225
   TGG CCG CAA CAG CAG GGG CGA TAT CCT CTG ACG TAT CAT CTT TTT TTG 3029
   Trp Pro Gln Gln Gln Gly Arg Tyr Pro Leu Thr Tyr His Leu Phe Leu
   230 235 240 245
   GGA GTG ATT GCT CGT GAG TAAACGTTAT TTTGTCACCG GAACGGATAC 3077
   Gly Val Ile Ala Arg Glu
   250
   CGAAGTGGGG AAAACTGTCG CCAGTTGTGC ACTTTTACAA GCCGCAAAGG CAGCAGGCTA 3137
   CCGGACGGCA GGTTATAAAC CGGTCGCCTC TGGCAGCGAA AAGACCCCGG AAGGTTTACG 3197
   CAATAGCGAC GCGCTGGCGT TACAGCGCAA CAGCAGCCTG CAGCTGGATT ACGCAACAGT 3257
   AAATCCTTAC ACCTTCGCAG AACCCACTTC GCCGCACATC ATCAGCGCGC AAGAGGGCAG 3317
   ACCGATAGAA TCATTGGTAA TGAGCGCCGG ATTACGCGCG CTTGAACAAC AGGCTGACTG 3377
   GGTGTTAGTG GAAGGTGCTG GCGGCTGGTT TACGCCGCTT TCTGACACTT TCACTTTTGC 3437
   AGATTGGGTA ACACAGGAAC AACTGCCGGT GATACTGGTA GTTGGTGTGA AACTCGGCTG 3497
   TATTAATCAC GCGATGTTGA CTGCACAGGT AATACAACAC GCCGGACTGA CTCTGGCGGG 3557
   TTGGGTGGCG AACGATGTTA CGCCTCCGGG AAAACGTCAC GCTGAATATA TGACCACGCT 3617
   CACCCGCATG ATTCCCGCGC CGCTGCTGGG AGAGATCCCC TGGCTTGCAG AAAATCCAGA 3677
   AAATGCGGCA ACCGGAAAGT ACATAAACCT TGCCTTCGTC GACGCGTCGA CTCTAGGGTT 3737
   TACAAGTCGA TT ATG ACA ACG GAC GAT CTT GCC TTT GAC CAA CGC CAT 3785
   Met Thr Thr Asp Asp Leu Ala Phe Asp Gln Arg His
   1 5 10
   ATC TGG CAC CCA TAC ACA TCC ATG ACC TCC CCT CTG CCG GTT TAT CCG 3833
   Ile Trp His Pro Tyr Thr Ser Met Thr Ser Pro Leu Pro Val Tyr Pro
   15 20 25
   GTG GTG AGC GCC GAA GGT TGC GAG CTG ATT TTG TCT GAC GGC AGA CGC 3881
   Val Val Ser Ala Glu Gly Cys Glu Leu Ile Leu Ser Asp Gly Arg Arg
   30 35 40
   CTG GTT GAC GGT ATG TCG TCC TGG TGG GCG GCG ATC CAC GGC TAC AAT     3929
   Leu Val Asp Gly Met Ser Ser Trp Trp Ala Ala Ile His Gly Tyr Asn
    45                 50                  55                  60
   CAC CCG CAG CTT AAT GCG GCG ATG AAG TCG CAA ATT GAT GCC ATG TCG     3977
   His Pro Gln Leu Asn Ala Ala Met Lys Ser Gln Ile Asp Ala Met Ser
               65                  70                  75
   CAT GTG ATG TTT GGC GGT ATC ACC CAT GCG CCA GCC ATT GAG CTG TGC     4025
   His Val Met Phe Gly Gly Ile Thr His Ala Pro Ala Ile Glu Leu Cys
           80                  85                  90
   CGC AAA CTG GTG GCG ATG ACG CCG CAA CCG CTG GAG TGC GTT TTT CTC     4073
   Arg Lys Leu Val Ala Met Thr Pro Gln Pro Leu Glu Cys Val Phe Leu
        95                 100                 105
   GCG GAC TCC GGT TCC GTA GCG GTG GAA GTG GCG ATG AAA ATG GCG TTG     4121
   Ala Asp Ser Gly Ser Val Ala Val Glu Val Ala Met Lys Met Ala Leu
       110                 115                 120
   CAG TAC TGG CAA GCC AAA GGC GAA GCG CGC CAG CGT TTT CTG ACC TTC     4169
   Gln Tyr Trp Gln Ala Lys Gly Glu Ala Arg Gln Arg Phe Leu Thr Phe
   125                 130                 135                 140
   CGC AAT GGT TAT CAT GGC GAT ACC TTT GGC GCG ATG TCG GTG TGC GAT     4217
   Arg Asn Gly Tyr His Gly Asp Thr Phe Gly Ala Met Ser Val Cys Asp
               145                 150                 155
   CCG GAT AAC TCA ATG CAC AGT CTG TGG AAA GGC TAC CTG CCA GAA AAC     4265
   Pro Asp Asn Ser Met His Ser Leu Trp Lys Gly Tyr Leu Pro Glu Asn
           160                 165                 170
   CTG TTT GCT CCC GCC CCG CAA AGC CGC ATG GAT GGC GAA TGG GAT GAG     4313
   Leu Phe Ala Pro Ala Pro Gln Ser Arg Met Asp Gly Glu Trp Asp Glu
       175                 180                 185
   CGC GAT ATG GTG GGC TTT GCC CGC CTG ATG GCG GCG CAT CGT CAT GAA     4361
   Arg Asp Met Val Gly Phe Ala Arg Leu Met Ala Ala His Arg His Glu
       190                 195                 200
   ATC GCG GCG GTG ATC ATT GAG CCG ATT GTC CAG GGC GCA GGC GGG ATG     4409
   Ile Ala Ala Val Ile Ile Glu Pro Ile Val Gln Gly Ala Gly Gly Met
   205                 210                 215                 220
   CGC ATG TAC CAT CCG GAA TGG TTA AAA CGA ATC CGC AAA ATA TGC GAT     4457
   Arg Met Tyr His Pro Glu Trp Leu Lys Arg Ile Arg Lys Ile Cys Asp
               225                 230                 235
   CGC GAA GGT ATC TTG CTG ATT GCC GAC GAG ATC GCC ACT GGA TTT GGT     4505
   Arg Glu Gly Ile Leu Leu Ile Ala Asp Glu Ile Ala Thr Gly Phe Gly
           240                 245                 250
   CGT ACC GGG AAA CTG TTT GCC TGT GAA CAT GCA GAA ATC GCG CCG GAC     4553
   Arg Thr Gly Lys Leu Phe Ala Cys Glu His Ala Glu Ile Ala Pro Asp
       255                 260                 265
   ATT TTG TGC CTC GGT AAA GCC TTA ACC GGC GGC ACA ATG ACC CTT TCC     4601
   Ile Leu Cys Leu Gly Lys Ala Leu Thr Gly Gly Thr Met Thr Leu Ser
       270                 275                 280
   GCC ACA CTC ACC ACG CGC GAG GTT GCA GAA ACC ATC AGT AAC GGT GAA     4649
   Ala Thr Leu Thr Thr Arg Glu Val Ala Glu Thr Ile Ser Asn Gly Glu
   285                 290                 295                 300
   GCC GGT TGC TTT ATG CAT GGG CCA ACT TTT ATG GGC AAT CCG CTG GCC      4697
   Ala Gly Cys Phe Met His Gly Pro Thr Phe Met Gly Asn Pro Leu Ala
               305                 310                 315
   TGC GCG GCA GCA AAC GCC AGC CTG GCG ATT CTC GAA TCT GGC GAC TGG      4745
   Cys Ala Ala Ala Asn Ala Ser Leu Ala Ile Leu Glu Ser Gly Asp Trp
           320                 325                 330
   CAG CAA CAG GTG GCG GAT ATT GAA GTA CAG CTG CGC GAG CAA CTT GCC      4793
   Gln Gln Gln Val Ala Asp Ile Glu Val Gln Leu Arg Glu Gln Leu Ala
       335                 340                 345
   CCC GCC CGT GAT GCC GAA ATG GTT GCC GAT GTG CGC GTA CTG GGG GCC      4841
   Pro Ala Arg Asp Ala Glu Met Val Ala Asp Val Arg Val Leu Gly Ala
   350                 355                 360
   ATT GGC GTG GTC GAA ACC ACT CAT CCG GTG AAT ATG GCG GCG CTG CAA      4889
   Ile Gly Val Val Glu Thr Thr His Pro Val Asn Met Ala Ala Leu Gln
   365                 370                 375                 380
   AAA TTC TTT GTC GAA CAG GGT GTC TGG ATC CGG CCT TTT GGC AAA CTG      4937
   Lys Phe Phe Val Glu Gln Gly Val Trp Ile Arg Pro Phe Gly Lys Leu
                   385                 390                 395
   ATT TAC CTG ATG CCG CCC TAT ATT ATT CTC CCG CAA CAG TTG CAG CGT      4985
   Ile Tyr Leu Met Pro Pro Tyr Ile Ile Leu Pro Gln Gln Leu Gln Arg
               400                 405                 410
   CTG ACC GCA GCG GTT AAC CGC GCG GTA CAG GAT GAA ACA TTT TTT TGC      5033
   Leu Thr Ala Ala Val Asn Arg Ala Val Gln Asp Glu Thr Phe Phe Cys
           415                 420                 425
   CAA TAACGAGAAG TCCGCGTGAG GGTTTCTGGC TACACTTTCT GCAAACAAGA           5086
   Gln
   430
   AAGGAGGGTT C ATG AAA CTC ATC AGT AAC GAT CTG CGC GAT GGC GAT AAA     5136
                Met Lys Leu Ile Ser Asn Asp Leu Arg Asp Gly Asp Lys
                1               5                   10
   TTG CCG CAT CGT CAT GTC TTT AAC GGC ATG GGT TAC GAT GGC GAT AAT      5184
   Leu Pro His Arg His Val Phe Asn Gly Met Gly Tyr Asp Gly Asp Asn
       15                  20                  25
   ATT TCA CCG CAT CTG GCG TGG GAT GAT GTT CCT GCG GGA ACG AAA AGT      5232
   Ile Ser Pro His Leu Ala Trp Asp Asp Val Pro Ala Gly Thr Lys Ser
   30                  35                  40                  45
   TTT GTT GTC ACC TGC TAC GAC CCG GAT GCG CCA ACC GGC TCC GGC TGG      5280
   Phe Val Val Thr Cys Tyr Asp Pro Asp Ala Pro Thr Gly Ser Gly Trp
                   50                  55                  60
   TGG CAC TGG GTA GTT GTT AAC TTA CCC GCT GAT ACC CGC GTA TTA CCG      5328
   Trp His Trp Val Val Val Asn Leu Pro Ala Asp Thr Arg Val Leu Pro
               65                  70                  75
   CAA GGG TTT GGC TCT GGT CTG GTA GCA ATG CCA GAC GGC GTT TTG CAG      5376
   Gln Gly Phe Gly Ser Gly Leu Val Ala Met Pro Asp Gly Val Leu Gln
           80                  85                  90
   ACG CGT ACC GAC TTT GGT AAA ACC GGG TAC GAT GGC GCA GCA CCG CCG      5424
   Thr Arg Thr Asp Phe Gly Lys Thr Gly Tyr Asp Gly Ala Ala Pro Pro
       95                  100                 105
   AAA GGC GAA ACT CAT CGC TAC ATT TTT ACC GTT CAC GCG CTG GAT ATA        5472
   Lys Gly Glu Thr His Arg Tyr Ile Phe Thr Val His Ala Leu Asp Ile
   110                 115                 120                 125
   GAA CGT ATT GAT GTC GAT GAA GGT GCC AGC GGC GCG ATG GTC GGG TTT        5520
   Glu Arg Ile Asp Val Asp Glu Gly Ala Ser Gly Ala Met Val Gly Phe
                   130                 135                 140
   AAC GTT CAT TTC CAC TCT CTG GCA AGC GCC TCG ATT ACT GCG ATG TTT        5568
   Asn Val His Phe His Ser Leu Ala Ser Ala Ser Ile Thr Ala Met Phe
               145                 150                 155
   AGT TAATCACTCT GCCAGATGGC GCAATGCCAT CTGGTATCAC TTAAAGGTAT             5621
   Ser
   TAAAAACAAC TTTTTGTCTT TTTACCTTCC CGTTTCGCTC AAGTTAGTAT AAAAAAGCAG      5681
   GCTTCAACGG ATTCATTTTT CTATTTCATA GCCCGGAGCA ACCTGTGAAC ACATTTTCAG      5741
   TTTCCCGTCT GGCGCTGGCA TTGGCTTTTG GCGTGACGCT GACCGCCTGT AGCTCAACCC      5801
   CGCCCGATCA ACGTCCTTCT GATCAAACCG CGCCTGGTAC CGAGCTCGAA TTCCTGCAGG      5861
   CATGCAAGCT T                                                           5872
(2) INFORMATION ZU SEQ ID NO: 2:
   (i) SEQUENZ CHARAKTERISTIKA:
      (A) LÄNGE: 346 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Ala His Arg Pro Arg Trp Thr Leu Ser Gln Val Thr Glu Leu Phe
   1 5 10 15
   Glu Lys Pro Leu Leu Asp Leu Leu Phe Glu Ala Gln Gln Val His Arg
   20 25 30
   Gln His Phe Asp Pro Arg Gln Val Gln Val Ser Thr Leu Leu Ser Ile
   35 40 45
   Lys Thr Gly Ala Cys Pro Glu Asp Cys Lys Tyr Cys Pro Gln Ser Ser
   50 55 60
   Arg Tyr Lys Thr Gly Leu Glu Ala Glu Arg Leu Met Glu Val Glu Gln
   65 70 75 80
   Val Leu Glu Ser Ala Arg Lys Ala Lys Ala Ala Gly Ser Thr Arg Phe
   85 90 95
   Cys Met Gly Ala Ala Trp Lys Asn Pro His Glu Arg Asp Met Pro Tyr
   100 105 110
   Leu Glu Gln Met Val Gln Gly Val Lys Ala Met Gly Leu Glu Ala Cys
   115 120 125
   Met Thr Leu Gly Thr Leu Ser Glu Ser Gln Ala Gln Arg Leu Ala Asn
   130 135 140
   Ala Gly Leu Asp Tyr Tyr Asn His Asn Leu Asp Thr Ser Pro Glu Phe
   145 150 155 160
   Tyr Gly Asn Ile Ile Thr Thr Arg Thr Tyr Gln Glu Arg Leu Asp Thr
   165 170 175
   Leu Glu Lys Val Arg Asp Ala Gly Ile Lys Val Cys Ser Gly Gly Ile
   180 185 190
   Val Gly Leu Gly Glu Thr Val Lys Asp Arg Ala Gly Leu Leu Leu Gln
   195 200 205
   Leu Ala Asn Leu Pro Thr Pro Pro Glu Ser Val Pro Ile Asn Met Leu
   210 215 220
   Val Lys Val Lys Gly Thr Pro Leu Ala Asp Asn Asp Asp Val Asp Ala
   225 230 235 240
   Phe Asp Phe Ile Arg Thr Ile Ala Val Ala Arg Ile Met Met Pro Thr
   245 250 255
   Ser Tyr Val Arg Leu Ser Ala Gly Arg Glu Gln Met Asn Glu Gln Thr
   260 265 270
   Gln Ala Met Cys Phe Met Ala Gly Ala Asn Ser Ile Phe Tyr Gly Cys
   275 280 285
   Lys Leu Leu Thr Thr Pro Asn Pro Glu Glu Asp Lys Asp Leu Gln Leu
   290 295 300
   Phe Arg Lys Leu Gly Leu Asn Pro Gln Gln Thr Ala Val Leu Ala Gly
   305 310 315 320
   Asp Asn Glu Gln Gln Gln Arg Leu Glu Gln Ala Leu Met Thr Pro Asp
   325 330 335
   Thr Asp Glu Tyr Tyr Asn Ala Ala Ala Leu
   340 345
(2) INFORMATION ZU SEQ ID NO: 3:
   (i) SEQUENZ CHARAKTERISTIKA:
      (A) LÄNGE: 251 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 3:
(2) INFORMATION ZU SEQ ID NO: 4:
   (i) SEQUENZ CHARAKTERISTIKA:
      (A) LÄNGE: 429 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 4:
   Met Thr Thr Asp Asp Leu Ala Phe Asp Gln Arg His Ile Trp His Pro
   1 5 10 15
   Tyr Thr Ser Met Thr Ser Pro Leu Pro Val Tyr Pro Val Val Ser Ala
   20 25 30
   Glu Gly Cys Glu Leu Ile Leu Ser Asp Gly Arg Arg Leu Val Asp Gly
   35 40 45
   Met Ser Ser Trp Trp Ala Ala Ile His Gly Tyr Asn His Pro Gln Leu
   50 55 60
   Asn Ala Ala Met Lys Ser Gln Ile Asp Ala Met Ser His Val Met Phe
   65 70 75 80
   Gly Gly Ile Thr His Ala Pro Ala Ile Glu Leu Cys Arg Lys Leu Val
   85 90 95
   Ala Met Thr Pro Gln Pro Leu Glu Cys Val Phe Leu Ala Asp Ser Gly
   100 105 110
   Ser Val Ala Val Glu Val Ala Met Lys Met Ala Leu Gln Tyr Trp Gln
   115 120 125
   Ala Lys Gly Glu Ala Arg Gln Arg Phe Leu Thr Phe Arg Asn Gly Tyr
   130 135 140
   His Gly Asp Thr Phe Gly Ala Met Ser Val Cys Asp Pro Asp Asn Ser
   145 150 155 160
   Met His Ser Leu Trp Lys Gly Tyr Leu Pro Glu Asn Leu Phe Ala Pro
   165 170 175
   Ala Pro Gln Ser Arg Met Asp Gly Glu Trp Asp Glu Arg Asp Met Val
   180 185 190
   Gly Phe Ala Arg Leu Met Ala Ala His Arg His Glu Ile Ala Ala Val
   195 200 205
   Ile Ile Glu Pro Ile Val Gln Gly Ala Gly Gly Met Arg Met Tyr His
   210 215 220
   Pro Glu Trp Leu Lys Arg Ile Arg Lys Ile Cys Asp Arg Glu Gly Ile
   225 230 235 240
   Leu Leu Ile Ala Asp Glu Ile Ala Thr Gly Phe Gly Arg Thr Gly Lys
   245 250 255
   Leu Phe Ala Cys Glu His Ala Glu Ile Ala Pro Asp Ile Leu Cys Leu
   260 265 270
   Gly Lys Ala Leu Thr Gly Gly Thr Met Thr Leu Ser Ala Thr Leu Thr
   275 280 285
   Thr Arg Glu Val Ala Glu Thr Ile Ser Asn Gly Glu Ala Gly Cys Phe
   290 295 300
   Met His Gly Pro Thr Phe Met Gly Asn Pro Leu Ala Cys Ala Ala Ala
   305 310 315 320
   Asn Ala Ser Leu Ala Ile Leu Glu Ser Gly Asp Trp Gln Gln Gln Val
   325 330 335
   Ala Asp Ile Glu Val Gln Leu Arg Glu Gln Leu Ala Pro Ala Arg Asp
   340 345 350
   Ala Glu Met Val Ala Asp Val Arg Val Leu Gly Ala Ile Gly Val Val
   355 360 365
   Glu Thr Thr His Pro Val Asn Met Ala Ala Leu Gln Lys Phe Phe Val
   370 375 380
   Glu Gln Gly Val Trp Ile Arg Pro Phe Gly Lys Leu Ile Tyr Leu Met
   385 390 395 400
   Pro Pro Tyr Ile Ile Leu Pro Gln Gln Leu Gln Arg Leu Thr Ala Ala
   405 410 415
   Val Asn Arg Ala Val Gln Asp Glu Thr Phe Phe Cys Gln
   420 425
(2) INFORMATION ZU SEQ ID NO: 5:
   (i) SEQUENZ CHARAKTERISTIKA:
      (A) LÄNGE: 158 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 5:
   Met Lys Leu Ile Ser Asn Asp Leu Arg Asp Gly Asp Lys Leu Pro His
   1 5 10 15
   Arg His Val Phe Asn Gly Met Gly Tyr Asp Gly Asp Asn Ile Ser Pro
   20 25 30
   His Leu Ala Trp Asp Asp Val Pro Ala Gly Thr Lys Ser Phe Val Val
   35 40 45
   Thr Cys Tyr Asp Pro Asp Ala Pro Thr Gly Ser Gly Trp Trp His Trp
   50 55 60
   Val Val Val Asn Leu Pro Ala Asp Thr Arg Val Leu Pro Gln Gly Phe
   65 70 75 80
   Gly Ser Gly Leu Val Ala Met Pro Asp Gly Val Leu Gln Thr Arg Thr
   85 90 95
   Asp Phe Gly Lys Thr Gly Tyr Asp Gly Ala Ala Pro Pro Lys Gly Glu
   100 105 110
   Thr His Arg Tyr Ile Phe Thr Val His Ala Leu Asp Ile Glu Arg Ile
   115 120 125
   Asp Val Asp Glu Gly Ala Ser Gly Ala Met Val Gly Phe Asn Val His
   130 135 140
   Phe His Ser Leu Ala Ser Ala Ser Ile Thr Ala Met Phe Ser
   145 150 155
(2) INFORMATION ZU SEQ ID NO: 6:
   (i) SEQUENZ CHARAKTERISTIKA:
      (A) LÄNGE: 5872 Basenpaare
      (B) ART: Nukleinsäure
      (C) STRANGFORM: Doppel
      (D) TOPOLOGIE: linear
   (ii) ART DES MOLEKÜLS: DNS (genomisch)
   (iii) HYPOTHETISCH: NEIN
   (vi) URSPRÜNLICHE HERKUNFT:
      (A) ORGANISMUS: Escherichia coli
      (B) STAMM: DSM498
   (vii) UNMITTELBARE HERKUNFT:
      (B) CLON: pBO30A15-9
   (ix) MERKMALE:
      (A) NAME/SCHLÜSSEL: CDS
      (B) LAGE: 1152..2308
      (C) ART DER ERMITTLUNG: experimentell
      (D) SONSTIGE ANGABEN: /codon_start= 1154
         /EC_numher= 2.3.1.47
         /product= "KAPA synthase"
         /evidence= EXPERIMENTAL
         /gene= "bioF"
         /number= 2
         /standard_name= "8-Amino-7-oxononanoate synthase"
   (ix) MERKMALE:
      (A) NAME/SCHLÜSSEL: CDS
      (B) LAGE: 3043..3753
      (C) ART DER ERMITTLUNG: experimentell
      (D) SONSTIGE ANGABEN: /codon_start= 3043
         /EC_numher= 6.3.3.3
         /product= "DTB Synthase"
         /evidence= EXPERIMENTAL
         /gene= "bioD"
         /number= 4
         /standard_name= "Dethiobiotin Synthase"
   (ix) MERKMALE:
      (A) NAME/SCHLÜSSEL: RBS
      (B) LAGE: 1141..1156
      (D) SONSTIGE ANGABEN= /standard_name· "bioF RBS"
   (ix) MERKMALE:
      (A) NAME/SCHLÜSSEL: RBS
      (B) LAGE: 3030..3045
      (D) SONSTIGE ANGABEN: /standard_name= "bioD RBS"
   (x) VERÖFFENTLICHUNGSINFORMATION:
      (H) DOCUMENTNUMMER: WO 87/01391 B1
      (I) ANMELDEDATUM: 26-AUG-1986
      (J) VERÖFFENTLICHUNGSTAG: 07-APR-1993
   (xi) SEQUENZBESCHREIBUNG: SEQ ID NO: 6:
   AAGCTTACTC CCCATCCCCC TGTTGACAAT TAATCATCGG CTCGTATAAT GTGTGGAATT 60
   GTGAGCGGAT AACAATTTCA CACAGGAAAC AGGATCGGTA CCTAAGGAGA CTAGTCATGG 120
   CTCACCGCCC ACGCTGGACA TTGTCGCAAG TCACAGAATT ATTTGAAAAA CCGTTGCTGG 180
   ATCTGCTGTT TGAAGCGCAG CAGGTGCATC GCCAGCATTT CGATCCTCGT CAGGTGCAGC 240
   TCAGCACGTT GCTGTCGATT AAGACCGGAG CTTGTCCGGA AGATTGCAAA TACTGCCCGC 300
   AAAGCTCGCG CTACAAAACC GGGCTGGAAG CCGAGCGGTT GATGGAAGTT GAACAGGTGC 360
   TGGAGTCGGC GCGCAAAGCG AAAGCGGCAG GATCGACGCG CTTCTGTATG GGCGCGGCGT 420
   GGAAGAATCC CCACGAACGC GATATGCCGT ACCTGGAACA AATGGTGCAG GGGGTAAAAG 480
   CGATGGGGCT GGAGGCGTGT ATGACGCTGG GCACGTTGAG TGAATCTCAG GCGCAGCGCC 540
   TCGCGAACGC CGGGCTGGAT TACTACAACC ACAACCTGGA CACCTCGCCG GAGTTTTACG 600
   GCAATATCAT CACCACACGC ACTTATCAGG AACGCCTCGA TACGCTGGAA AAAGTGCGCG     660
   ATGCCGGGAT CAAAGTCTGT TCTGGCGGCA TTGTGGGCTT AGGCGAAACG GTAAAAGATC     720
   GCGCCGGATT ATTGCTGCAA CTGGCAAACC TGCCGACGCC GCCGGAAAGC GTGCCAATCA     780
   ACATGCTGGT GAAGGTGAAA GGCACGCCGC TTGCCGATAA CGATGATGTC GATGCCTTTG     840
   ATTTTATTCG CACCATTGCG GTCGCGCGGA TCATGATGCC AACCTCTTAC GTGCGCCTTT     900
   CTGCCGGACG CGAGCAGATG AACGAACAGA CTCAGGCGAT GTGCTTTATG GCAGGCGCAA     960
   ACTCGATTTT CTACGGTTGC AAACTGCTGA CCACGCCGAA TCCGGAAGAA GATAAAGACC    1020
   TGCAACTGTT CCGCAAACTG GGGCTAAATC CGCAGCAAAC TGCCGTGCTG GCAGGGGATA    1080
   ACGAACAACA GCAACGTCTT GAACAGGCGC TGATGACCCC GGACACCGAC GAATATTACA    1140
   ACGCGGCAGC ATT ATG AGC TGG CAG GAG AAA ATC AAC GCG GCG CTC GAT       1189
                  Met Ser Trp Gln Glu Lys Ile Asn Ala Ala Leu Asp
                   1               5                  10
   GCG CGG CGT GCT GCC GAT GCC CTG CGT CGC CGT TAT CCG GTG GCG CAA      1237
   Ala Arg Arg Ala Ala Asp Ala Leu Arg Arg Arg Tyr Pro Val Ala Gln
           15                  20                  25
   GGA GCC GGA CGC TGG CTG GTG GCG GAT GAT CGC CAG TAT CTG AAC TTT      1285
   Gly Ala Gly Arg Trp Leu Val Ala Asp Asp Arg Gln Tyr Leu Asn Phe
       30                  35                  40
   TCC AGT AAC GAT TAT TTA GGT TTA AGC CAT CAT CCG CAA ATT ATC CGT      1333
   Ser Ser Asn Asp Tyr Leu Gly Leu Ser His His Pro Gln Ile Ile Arg
   45                  50                  55                  60
   GCC TGG CAG CAG GGG GCG GAG CAA TTT GGC ATC GGT AGC GGC GGC TCC      1381
   Ala Trp Gln Gln Gly Ala Glu Gln Phe Gly Ile Gly Ser Gly Gly Ser
                   65                  70                  75
   GGT CAC GTC AGC GGT TAT AGC GTG GTG CAT CAG GCA CTG GAA GAA GAG      1429
   Gly His Val Ser Gly Tyr Ser Val Val His Gln Ala Leu Glu Glu Glu
               80                  85                  90
   CTG GCC GAG TGG CTT GGC TAT TCG CGG GCA CTG CTG TTT ATC TCT GGT      1477
   Leu Ala Glu Trp Leu Gly Tyr Ser Arg Ala Leu Leu Phe Ile Ser Gly
           95                  100                 105
   TTC GCC GCT AAT CAG GCA GTT ATT GCC GCG ATG ATG GCG AAA GAG GAC      1525
   Phe Ala Ala Asn Gln Ala Val Ile Ala Ala Met Met Ala Lys Glu Asp
       110                 115                 120
   CGT ATT GCT GCC GAC CGG CTT AGC CAT GCC TCA TTG CTG GAA GCT GCC      1573
   Arg Ile Ala Ala Asp Arg Leu Ser His Ala Ser Leu Leu Glu Ala Ala
   125                 130                 135                 140
   AGT TTA AGC CCG TCG CAG CTT CGC CGT TTT GCT CAT AAC GAT GTC ACT      1621
   Ser Leu Ser Pro Ser Gln Leu Arg Arg Phe Ala His Asn Asp Val Thr
                   145                 150                 155
   CAT TTG GCG CGA TTG CTT GCT TCC CCC TGT CCG GGG CAG CAA ATG GTG      1669
   His Leu Ala Arg Leu Leu Ala Ser Pro Cys Pro Gly Gln Gln Met Val
               160                 165                 170
   GTG ACA GAA GGC GTG TTC AGC ATG GAC GGC GAT AGT GCG CCA CTG GCG      1717
   Val Thr Glu Gly Val Phe Ser Met Asp Gly Asp Ser Ala Pro Leu Ala
   175 180 185
   GAA ATC CAG CAG GTA ACG CAA CAG CAC AAT GGC TGG TTG ATG GTC GAT 1765
   Glu Ile Gln Gln Val Thr Gln Gln His Asn Gly Trp Leu Met Val Asp
   190 195 200
   GAT GCC CAC GGC ACG GGC GTT ATC GGG GAG CAG GGG CGC GGC AGC TGC 1813
   Asp Ala His Gly Thr Gly Val Ile Gly Glu Gln Gly Arg Gly Ser Cys
   205 210 215 220
   TGG CTG CAA AAG GTA AAA CCA GAA TTG CTG GTA GTG ACT TTT GGC AAA 1861
   Trp Leu Gln Lys Val Lys Pro Glu Leu Leu Val Val Thr Phe Gly Lys
   225 230 235
   GGA TTT GGC GTC AGC GGG GCA GCG GTG CTT TGC TCC AGT ACG GTG GCG 1909
   Gly Phe Gly Val Ser Gly Ala Ala Val Leu Cys Ser Ser Thr Val Ala
   240 245 250
   GAT TAT CTG CTG CAA TTC GCC CGC CAC CTT ATC TAC AGC ACC AGT ATG 1957
   Asp Tyr Leu Leu Gln Phe Ala Arg His Leu Ile Tyr Ser Thr Ser Met
   255 260 265
   CCG CCC GCT CAG GCG CAG GCA TTA CGT GCG TCG CTG GCG GTC ATT CGC 2005
   Pro Pro Ala Gln Ala Gln Ala Leu Arg Ala Ser Leu Ala Val Ile Arg
   270 275 280
   AGT GAT GAG GGT GAT GCA CGG CGC GAA AAA CTG GCG GCA CTC ATT ACG 2053
   Ser Asp Glu Gly Asp Ala Arg Arg Glu Lys Leu Ala Ala Leu Ile Thr
   285 290 295 300
   CGT TTT CGT GCC GGA GTA CAG GAT TTG CCG TTT ACG CTT GCT GAT TCA 2101
   Arg Phe Arg Ala Gly Val Gln Asp Leu Pro Phe Thr Leu Ala Asp Ser
   305 310 315
   TGC AGC GCC ATC CAG CCA TTG ATT GTC GGT GAT AAC AGC CGT GCG TTA 2149
   Cys Ser Ala Ile Gln Pro Leu Ile Val Gly Asp Asn Ser Arg Ala Leu
   320 325 330
   CAA CTG GCA GAA AAA CTG CGT CAG CAA GGC TGC TGG GTC ACG GCG ATT 2197
   Gln Leu Ala Glu Lys Leu Arg Gln Gln Gly Cys Trp Val Thr Ala Ile
   335 340 345
   CGC CCG CCA ACC GTA CCC GCT GGT ACT GCG CGA CTG CGC TTA ACG CTA 2245
   Arg Pro Pro Thr Val Pro Ala Gly Thr Ala Arg Leu Arg Leu Thr Leu
   350 355 360
   ACC GCT GCG CAT GAA ATG CAG GAT ATC GAC CGT CTG CTG GAG GTG CTG 2293
   Thr Ala Ala His Glu Met Gln Asp Ile Asp Arg Leu Leu Glu Val Leu
   365 370 375 380
   CAT GGC AAC GGT TAATAAACAA GCCATTGCAG CGGCATTTGG TCGGGCAGCC 2345
   His Gly Asn Gly
   385
   GCACACTATG AGCAACATGC AGATCTACAG CGCCAGAGTG CTGACGCCTT ACTGGCAATG 2405
   CTTCCACAGC GTAAATACAC CCACGTACTG GACGCGGGTT GTGGACCTGG CTGGATGAGC 2465
   CGCCACTGGC GGGAACGTCA CGCGCAGGTG ACGGCCTTAG ATCTCTCGCC GCCAATGCTT 2525
   GTTCAGGCAC GCCAGAAGGA TGCCGCAGAC CATTATCTGG CGGGAGATAT CGAATCCCTG 2585
   CCGTTAGCGA CTGCGACGTT CGATCTTGCA TGGAGCAATC TCGCAGTGCA GTGGTGCGCT 2645
   AATTTATCCA CGGCACTCCG CGAGCTGTAT CGGGTGGTGC GCCCCAAAGG CGTGGTCGCG    2705
   TTTACCACGC TGGTGCAGGG ATCGTTACCC GAACTGCATC AGGCGTGGCA GGCGGTGGAC    2765
   GAGCGTCCGC ATGCTAATCG CTTTTTACCG CCAGATGAAA TCGAACAGTC GCTGAACGGC    2825
   GTGCATTATC AACATCATAT TCAGCCCATC ACGCTGTGGT TTGATGATGC GCTCAGTGCC    2885
   ATGCGTTCGC TGAAAGGCAT CGGTGCCACG CATCTTCATG AAGGGCGCGA CCCGCGAATA    2945
   TTAACGCGTT CGCAGTTGCA GCGATTGCAA CTGGCCTGGC CGCAACAGCA GGGGCGATAT    3005
   CCTCTGACGT ATCATCTTTT TTTGGGAGTG ATTGCTC GTG AGT AAA CGT TAT TTT    3060
                                           Val Ser Lys Arg Tyr Phe
                                             1               5
   GTC ACC GGA ACG GAT ACC GAA GTG GGG AAA ACT GTC GCC AGT TGT GCA    3108
   Val Thr Gly Thr Asp Thr Glu Val Gly Lys Thr Val Ala Ser Cys Ala
               10                  15                  20
   CTT TTA CAA GCC GCA AAG GCA GCA GGC TAC CGG ACG GCA GGT TAT AAA    3156
   Leu Leu Gln Ala Ala Lys Ala Ala Gly Tyr Arg Thr Ala Gly Tyr Lys
           25                  30                  35
   CCG GTC GCC TCT GGC AGC GAA AAG ACC CCG GAA GGT TTA CGC AAT AGC    3204
   Pro Val Ala Ser Gly Ser Glu Lys Thr Pro Glu Gly Leu Arg Asn Ser
       40                  45                  50
   GAC GCG CTG GCG TTA CAG CGC AAC AGC AGC CTG CAG CTG GAT TAC GCA    3252
   Asp Ala Leu Ala Leu Gln Arg Asn Ser Ser Leu Gln Leu Asp Tyr Ala
   55                  60                  65                  70
   ACA GTA AAT CCT TAC ACC TTC GCA GAA CCC ACT TCG CCG CAC ATC ATC    3300
   Thr Val Asn Pro Tyr Thr Phe Ala Glu Pro Thr Ser Pro His Ile Ile
                   75                  80                  85
   AGC GCG CAA GAG GGC AGA CCG ATA GAA TCA TTG GTA ATG AGC GCC GGA    3348
   Ser Ala Gln Glu Gly Arg Pro Ile Glu Ser Leu Val Met Ser Ala Gly
               90                  95                  100
   TTA CGC GCG CTT GAA CAA CAG GCT GAC TGG GTG TTA GTG GAA GGT GCT    3396
   Leu Arg Ala Leu Glu Gln Gln Ala Asp Trp Val Leu Val Glu Gly Ala
           105                 110                 115
   GGC GGC TGG TTT ACG CCG CTT TCT GAC ACT TTC ACT TTT GCA GAT TGG    3444
   Gly Gly Trp Phe Thr Pro Leu Ser Asp Thr Phe Thr Phe Ala Asp Trp
       120                 125                 130
   GTA ACA CAG GAA CAA CTG CCG GTG ATA CTG GTA GTT GGT GTG AAA CTC    3492
   Val Thr Gln Glu Gln Leu Pro Val Ile Leu Val Val Gly Val Lys Leu
   135                 140                 145                 150
   GGC TGT ATT AAT CAC GCG ATG TTG ACT GCA CAG GTA ATA CAA CAC GCC    3540
   Gly Cys Ile Asn His Ala Met Leu Thr Ala Gln Val Ile Gln His Ala
                   155                 160                 165
   GGA CTG ACT CTG GCG GGT TGG GTG GCG AAC GAT GTT ACG CCT CCG GGA    3588
   Gly Leu Thr Leu Ala Gly Trp Val Ala Asn Asp Val Thr Pro Pro Gly
               170                 175                 180
   AAA CGT CAC GCT GAA TAT ATG ACC ACG CTC ACC CGC ATG ATT CCC GCG    3636
   Lys Arg His Ala Glu Tyr Met Thr Thr Leu Thr Arg Met Ile Pro Ala
           185                 190                 195
   CCG CTG CTG GGA GAG ATC CCC TGG CTT GCA GAA AAT CCA GAA AAT GCG 3684
   Pro Leu Leu Gly Glu Ile Pro Trp Leu Ala Glu Asn Pro Glu Asn Ala
   200 205 210
   GCA ACC GGA AAG TAC ATA AAC CTT GCC TTC GTC GAC GCG TCG ACT CTA 3732
   Ala Thr Gly Lys Tyr Ile Asn Leu Ala Phe Val Asp Ala Ser Thr Leu
   215 220 225 230
   GGG TTT ACA AGT CGA TTA TGACAACGGA CGATCTTGCC TTTGACCAAC 3780
   Gly Phe Thr Ser Arg Leu
   235
   GCCATATCTG GCACCCATAC ACATCCATGA CCTCCCCTCT GCCGGTTTAT CCGGTGGTGA 3840
   GCGCCGAAGG TTGCGAGCTG ATTTTGTCTG ACGGCAGACG CCTGGTTGAC GGTATGTCGT 3900
   CCTGGTGGGC GGCGATCCAC GGCTACAATC ACCCGCAGCT TAATGCGGCG ATGAAGTCGC 3960
   AAATTGATGC CATGTCGCAT GTGATGTTTG GCGGTATCAC CCATGCGCCA GCCATTGAGC 4020
   TGTGCCGCAA ACTGGTGGCG ATGACGCCGC AACCGCTGGA GTGCGTTTTT CTCGCGGACT 4080
   CCGGTTCCGT AGCGGTGGAA GTGGCGATGA AAATGGCGTT GCAGTACTGG CAAGCCAAAG 4140
   GCGAAGCGCG CCAGCGTTTT CTGACCTTCC GCAATGGTTA TCATGGCGAT ACCTTTGGCG 4200
   CGATGTCGGT GTGCGATCCG GATAACTCAA TGCACAGTCT GTGGAAAGGC TACCTGCCAG 4260
   AAAACCTGTT TGCTCCCGCC CCGCAAAGCC GCATGGATGG CGAATGGGAT GAGCGCGATA 4320
   TGGTGGGCTT TGCCCGCCTG ATGGCGGCGC ATCGTCATGA AATCGCGGCG GTGATCATTG 4380
   AGCCGATTGT CCAGGGCGCA GGCGGGATGC GCATGTACCA TCCGGAATGG TTAAAACGAA 4440
   TCCGCAAAAT ATGCGATCGC GAAGGTATCT TGCTGATTGC CGACGAGATC GCCACTGGAT 4500
   TTGGTCGTAC CGGGAAACTG TTTGCCTGTG AACATGCAGA AATCGCGCCG GACATTTTGT 4560
   GCCTCGGTAA AGCCTTAACC GGCGGCACAA TGACCCTTTC CGCCACACTC ACCACGCGCG 4620
   AGGTTGCAGA AACCATCAGT AACGGTGAAG CCGGTTGCTT TATGCATGGG CCAACTTTTA 4680
   TGGGCAATCC GCTGGCCTGC GCGGCAGCAA ACGCCAGCCT GGCGATTCTC GAATCTGGCG 4740
   ACTGGCAGCA ACAGGTGGCG GATATTGAAG TACAGCTGCG CGAGCAACTT GCCCCCGCCC 4800
   GTGATGCCGA AATGGTTGCC GATGTGCGCG TACTGGGGGC CATTGGCGTG GTCGAAACCA 4860
   CTCATCCGGT GAATATGGCG GCGCTGCAAA AATTCTTTGT CGAACAGGGT GTCTGGATCC 4920
   GGCCTTTTGG CAAACTGATT TACCTGATGC CGCCCTATAT TATTCTCCCG CAACAGTTGC 4980
   AGCGTCTGAC CGCAGCGGTT AACCGCGCGG TACAGGATGA AACATTTTTT TGCCAATAAC 5040
   GAGAAGTCCG CGTGAGGGTT TCTGGCTACA CTTTCTGCAA ACAAGAAAGG AGGGTTCATG 5100
   AAACTCATCA GTAACGATCT GCGCGATGGC GATAAATTGC CGCATCGTCA TGTCTTTAAC 5160
   GGCATGGGTT ACGATGGCGA TAATATTTCA CCGCATCTGG CGTGGGATGA TGTTCCTGCG 5220
   GGAACGAAAA GTTTTGTTGT CACCTGCTAC GACCCGGATG CGCCAACCGG CTCCGGCTGG 5280
   TGGCACTGGG TAGTTGTTAA CTTACCCGCT GATACCCGCC TATTACCGCA AGGGTTTGGC 5340
   TCTGGTCTGG TAGCAATGCC AGACGGCGTT TTGCAGACGC GTACCGACTT TGGTAAAACC    5400
   GGGTACGATG GCGCAGCACC GCCGAAAGGC GAAACTCATC GCTACATTTT TACCGTTCAC    5460
   GCGCTGGATA TAGAACGTAT TGATGTCGAT GAAGGTGCCA GCGGCGCGAT GGTCGGGTTT    5520
   AACGTTCATT TCCACTCTCT GGCAAGCGCC TCGATTACTG CGATGTTTAG TTAATCACTC    5580
   TGCCAGATGG CGCAATGCCA TCTGGTATCA CTTAAAGGTA TTAAAAACAA CTTTTTGTCT    5640
   TTTTACCTTC CCGTTTCGCT CAAGTTAGTA TAAAAAAGCA GGCTTCAACG GATTCATTTT    5700
   TCTATTTCAT AGCCCGGAGC AACCTGTGAA CACATTTTCA GTTTCCCGTC TGGCGCTGGC    5760
   ATTGGCTTTT GGCGTGACGC TGACCGCCTG TAGCTCAACC CCGCCCGATC AACGTCCTTC    5820
   TGATCAAACC GCGCCTGGTA CCGAGCTCGA ATTCCTGCAG GCATGCAAGC TT            5872
(2) INFORMATION ZU SEQ ID NO: 7:
   (i) SEOUENZ CHARAKTERISTIKA:
      (A) LÄNGE: 384 Aminosäuren
      (B) ART: Aminosäure
      (D) TOPOLOGIE: linear
   (ii) ART DES MOLEKÜLS: Protein
      (xi) SEQUENZBESCHREIBUNG: SEQ ID NO: 7:
(2) INFORMATION ZU SEQ ID NO: 8:
   (i) SEQUENZ CHARAKTERISTIKA:
      (A) LÄNGE: 236 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 8:
   Val Ser Lys Arg Tyr Phe Val Thr Gly Thr Asp Thr Glu Val Gly Lys
   1 5 10 15
   Thr Val Ala Ser Cys Ala Leu Leu Gln Ala Ala Lys Ala Ala Gly Tyr
   20 25 30
   Arg Thr Ala Gly Tyr Lys Pro Val Ala Ser Gly Ser Glu Lys Thr Pro
   35 40 45
   Glu Gly Leu Arg Asn Ser Asp Ala Leu Ala Leu Gln Arg Asn Ser Ser
   50 55 60
   Leu Gln Leu Asp Tyr Ala Thr Val Asn Pro Tyr Thr Phe Ala Glu Pro
   65 70 75 80
   Thr Ser Pro His Ile Ile Ser Ala Gln Glu Gly Arg Pro Ile Glu Ser
   85 90 95
   Leu Val Met Ser Ala Gly Leu Arg Ala Leu Glu Gln Gln Ala Asp Trp
   100 105 110
   Val Leu Val Glu Gly Ala Gly Gly Trp Phe Thr Pro Leu Ser Asp Thr
   115 120 125
   Phe Thr Phe Ala Asp Trp Val Thr Gln Glu Gln Leu Pro Val Ile Leu
   130 135 140
   Val Val Gly Val Lys Leu Gly Cys Ile Asn His Ala Met Leu Thr Ala
   145 150 155 160
   Gln Val Ile Gln His Ala Gly Leu Thr Leu Ala Gly Trp Val Ala Asn
   165 170 175
   Asp Val Thr Pro Pro Gly Lys Arg His Ala Glu Tyr Met Thr Thr Leu
   180 185 190
   Thr Arg Met Ile Pro Ala Pro Leu Leu Gly Glu Ile Pro Trp Leu Ala
   195 200 205
   Glu Asn Pro Glu Asn Ala Ala Thr Gly Lys Tyr Ile Asn Leu Ala Phe
   210 215 220
   Val Asp Ala Ser Thr Leu Gly Phe Thr Ser Arg Leu
   225 230 235
(2) INFORMATION ZU SEQ ID NO: 9:
   (i) SEQUENZ CHARAKTERISTIKA:
      (A) LÄNGE: 143 Basenpaare
      (B) ART: Nukleinsäure
      (C) STRANGFORM : Doppel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (A) ORGANISMUS: Escherichia coli
   (vii) UNMITTELBARE HERKUNFT:
      (B) CLON: pBO30
   (ix) MERKMALE:
      (A) NAME/SCHLÜSSEL: CDS
      (B) LAGE: 1..24
      (D) SONSTIGE ANGABEN: /partial
         /EC_number= 6.3.3.3
         /product= "Dethiobiotin Synthase"
         /gene= "bioD"
   (ix) MERKMALE:
      (A) NAME/SCHLÜSSEL: CDS
      (B) LAGE: 120..143
      (D) SONSTIGE ANGABEN: /partial
         /codon_start= 120
         /EC_number= 2.6.1.62
         /product= "DAPA Synthase"
         /gene= "bioA"
         /pseudo
   (ix) MERKMALE:
      (A) NAME/SCHLÜSSEL: RBS
      (B) LAGE: 111..122
      (D) SONSTIGE ANGABEN: /standard_name= "bioA RBS"
   (ix) MERKMALE:
      (A) NAME/SCHLÜSSEL: stem_loop
      (B) LAGE: 38..85
   (x) VERÖFFENTLICHUNGSINFORMATION:
      (H) DOCUMENTNUMMER: WO 87/01391 B1
      (I) ANMELDEDATUM: 26-AUG-1986
      (J) VERÖFFENTLICHUNGSTAG: 07-APR-1993
   (xi) SEQUENZBESCHREIBUNG: SEQ ID NO: 9:
   TAC ATA AAC CTT GCC TTG TTG TAGCCATTCT GTATTTGGTT AAATTGCGAG        51
   Tyr Ile Asn Leu Ala Leu Leu
    1               5
   CGAGATCGCG TCTTCGATTG ACTGCAATTT AACCCTCTAG AGTCGACTCT AGGGTTTACA  111
   AGTCGATT ATG ACA ACG GAC GAT CTT GCC TTT                           143
            Met Thr Thr Asp Asp Leu Ala Phe
             1               5
(2) INFORMATION ZU SEQ ID NO: 10:
   (i) SEQUENZ CHARAKTERISTIKA:
      (A) LÄNGE: 8 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 10:
(2) INFORMATION ZU SEQ ID NO: 11:
   (i) SEQUENZ CHARAKTERISTIKA:
      (A) LÄNGE: 7 Aminosäuren
      (B) ART : Aminosäure
      (D) TOPOLOGIE: linear
   (ii) ART DES MOLEKÜLS: Protein
   (xi) SEQUENZBESCHREIBUNG: SEQ ID NO: 11:
(2) INFORMATION ZU SEQ ID NO: 12:
   (i) SEQUENZ CHARAKTERISTIKA:
      (A) LÄNGE: 93 Basenpaare
      (B) ART: Nukleinsäure
      (C) STRANGFORM: Doppel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (A) ORGANISMUS: Escherichia coli
   (vii) UNMITTELBARE HERKUNFT:
      (B) CLON: pBO30A-9
   (ix) MERKMALE:
      (A) NAME/SCHLÜSSEL: CDS
      (B) LAGE: 1..24
      (D) SONSTIGE ANGABEN: /partial
         /codon_start= 1
         /EC_number= 6.3.3.3
         /product= "DTB Synthase"
         /gene= "bioD"
   (ix) MERKMALE:
      (A) NAME/SCHLÜSSEL: CDS
      (B) LAGE: 70..93
      (D) SONSTIGE ANGABEN: /partial
         /codon_start= 70
         /EC_number= 2.6.1.62
         /product= "DAPA Synthase"
         /gene= "bioA"
   (ix) MERKMALE:
      (A) NAME/SCHLÜSSEL: RBS
      (B) LAGE: 61..72
      (D) SONSTIGE ANGABEN: /standard_name= "bioA RBS"
   (x) VERÖFFENTLICHUNGSINFORMATION:
      (H) DOCUMENTNUMMER: WO 87/01391 B1
      (I) ANMELDEDATUM: 26-AUG-1986
      (J) VERÖFFENTLICHUNGSTAG: 07-APR-1993
   (xi) SEQUENZBESCHREIBUNG: SEQ ID NO: 12:
   TAC ATA AAC CTT GCC TTG TTG TAGCCATTCT GTATTTGGTT CGTCGACTCT     51
   Tyr Ile Asn Leu Ala Leu Leu
    1               5
   AGGGTTTACA AGTCGATT ATG ACA ACG GAC GAT CTT GCC TTT              93
                       Met Thr Thr Asp Asp Leu Ala Phe
                        1               5
(2) INFORMATION ZU SEQ ID NO: 13:
   (i) SEQUENZ CHARAKTERISTIKA:
      (A) LÄNZE: 8 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 13:
(2) INFORMATION ZU SEQ ID NO: 14:
   (i) SEQUENZ CHARAKTERISTIKA:
      (A) LÄNGE: 7 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 14:
(2) INFORMATION ZU SEQ ID NO: 15:
   (i) SEQUENZ CHARAKTERISTIKA:
      (A) LÄNGE: 77 Basenpaare
      (B) ART: Nukleinsäure
      (C) STRANGFORM: Doppel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (A) ORGANISMUS: Escherichia coli
   (vii) UNMITTELBARE HERKUNFT:
      (B) CLON: pBO30A-15
   (ix) MERKMALE:
      (A) NAME/SCHLÜSSEL: CDS
      (B) LAGE: 1..57
      (D) SONSTIGE ANGABEN: /partial
         /codon_start= 1
         /function= "altered 3'-end"
         /EC_number= 6.3.3.3
         /product= "DTB Synthase"
         /gene= "bioD"
   (ix) MERKMALE:
      (A) NAME/SCHLÜSSEL: CDS
      (B) LAGE: 54..77
      (D) SONSTIGE ANGABEN: /partial
         /codon_start= 54
         /EC_number= 2.6.1.62
         /product= "DAPA Synthase"
         /gene= "bioA"
   (ix) MERKMALE
      (A) NAME/SCHLÜSSEL: RBS
      (B) LAGE: 45..56
      (D) SONSTIGE ANGABEN: /standard_name= "bioA RBS"
   (x) VERÖFFENTLICHUNGSINFORMATION:
      (H) DOCUMENTNUMMER: WO 87/01391 B1
      (1) ANMELDEDATUM: 26-AUG-1986
      (J) VERÖFFENTLICHUNGSTAG: 07-APR-1993
   (xi) SEQUENZBESCHREIBUNG: SEQ ID NO: 15:
   TAC ATA AAC CTT GCC TTC GTC GAC GCG TCG ACT CTA GGG TTT ACA AGT      48
   Tyr Ile Asn Leu Ala Phe Val Asp Ala Ser Thr Leu Gly Phe Thr Ser
     1               5                  10                  15
   CGA TTA TGACAACGGA CGATCTTGCC TTT                                    77
   Arg Leu
(2) INFORMATION ZU SEQ ID NO: 16:
   (i) SEQUENZ CHARAKTERISTIKA:
      (A) LÄNGE: 18 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 16:
   Tyr Ile Asn Leu Ala Phe Val Asp Ala Ser Thr Leu Gly Phe Thr Ser
     1               5                  10                  15
   Arg Leu
(2) INFORMATION ZU SEQ ID NO: 17:
   (i) SEQUENZ CHARAKTERISTIKA:
      (A) LÄNGE: 125 Basenpaare
      (B) ART: Nukleinsäure
      (C) STRANGFORM: Doppel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (A) ORGANISMUS: Escherichia coli
   (vii) UNMITTELBARE HERKUNFT:
      (B) CLON: pBO30A-15/985E
   (ix) MERKMALE:
      (A) NAME/SCHLÜSSEL: -10_signal
      (B) LAGE: 45..49
      (D) SONSTIGE ANGABEN: /standard_name = "promoter ptac"
   (ix) MERKMALE:
      (A) NAME/SCHLÜSSEL: promoter
      (B) LAGE: 1..96
      (C) ART DER ERMITTLUNG: experimentell
      (D) SONSTIGE ANGABEN: /function= "promoter ptac"
         /evidence= EXPERIMENTAL
   (x) VERÖFFENTLICHUNGSINFORMATION:
      (H) DOCUMENTNUMMER: WO 87/01391 B1
      (I) ANMELDEDATUM: 26-AUG-1986
      (J) VERÖFFENTLICHUNGSTAG: 07-APR-1993
   (xi) SEQUENZBESCHREIBUNG: SEQ ID NO: 17:
   AAGCTTACTC CCCATCCCCC TGTTGACAAT TAATCATCGG CTCGTATAAT GTGTGGAATT    60
   GTGAGCGGAT AACAATTTCA CACAGGAAAC AGGATCGGTA CCTTAGGAGG TGACTAGTCA   120
   TGGCT                                                              125
(2) INFORMATION ZU SEQ ID NO: 18:
   (i) SEQUENZ CHARAKTERISTIKA:
      (A) LÄNGE: 126 Basenpaare
      (B) ART: Nukleinsäure
      (C) STRANGFORM: Doppel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (A) ORGANISMUS: Escherichia coli
   (vii) UNMITTELBARE HERKUNFT:
      (B) CLON: pBO30A-15/16
   (ix) MERKMALE:
      (A) NAME/SCHLÜSSEL: promoter
      (B) LAGE: 1..96
      (C) ART DER ERMITTLUNG: experimentell
      (D) SONSTIGE ANGABEN: /function= "promoter ptac"
         /evidence= EXPERIMENTAL
   (ix) MERKMALE:
      (A) NAME/SCHLÜSSEL: RBS
      (B) LAGE: 105..123
      (C) ART DER ERMITTLUNG: experimentell
      (D) SONSTIGE ANGABEN: /evidence= EXPERIMENTAL
         /standard_name= "bioB RBS no.16"
   (x) VERÖFFENTLICHUNGSINFORMATION:
      (H) DOCUMENTNUMMER: WO 87/01391 B1
      (I) ANMELDEDATUM: 26-AUG-1986
      (J) VERÖFFENTLICHUNGSTAG: 07-APR-1993
   (xi) SEQUENZBESCHREIBUNG: SEQ ID NO: 18:
   AAGCTTACTC CCCATCCCCC TGTTGACAAT TAATCATCGG CTCGTATAAT GTGTGGAATT    60
   GTGAGCGGAT AACAATTTCA CACAGGAAAC AGGATCGGTA CCTAAGGAGG TTTACTAGTC   120
   ATGGCT                                                              126
(2) INFORMATION ZU SEQ ID NO: 19:
   (i) SEQUENZ CHARAKTERISTIKA:
      (A) LÄNGE: 122 Basenpaare
      (B) ART: Nukleinsäure
      (C) STRANGFORM: Doppel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (A) ORGANISMUS: Escherichia coli
   (vii) UNMITTELBARE HERKUNFT:
      (B) CLON: pBO30A-15/9
   (ix) MERKMALE:
      (A) NAME/SCHLÜSSEL: promoter
      (B) LAGE: 1..96
      (C) ART DER ERMITTLUNG: experimentell
      (D) SONSTIGE ANGABEN: /function= "promoter ptac"
         /evidence= EXPERIMENTAL
      (ix) MERKMALE:
         (A) NAME/SCHLÜSSEL: RBS
         (B) LAGE: 105..119
         (C) ART DER ERMITTLUNG: experimentell
         (D) SONSTIGE ANGABEN: /evidence= EXPERIMENTAL
            /standard_name= "bioB RBS no. 9"
         (x) VERÖFFENTLICHUNGSINFORMATION:
            (H) DOCUMENTNUMMER: WO 87/01391 B1
            (I) ANMELDEDATUM: 26-AUG-1986
            (J) VERÖFFENTLICHUNGSTAG: 07-APR-1993
         (xi) SEQUENZBESCHREIBUNG: SEQ ID NO: 19:
   AAGCTTACTC CCCATCCCCC TGTTGACAAT TAATCATCGG CTCGTATAAT GTGTGGAATT     60
   GTGAGCGGAT AACAATTTCA CACAGGAAAC AGGATCGGTA CCTAAGGAGA CTAGTCATGG    120
   CT                                                                  122

## Beschreibung

Die Erfindung betrifft rekombinantes genetisches Material zur Expression der Gene des Biotin-Stoffwechselwegs von Enterobakterien, Mikroorganismen, die dieses rekombinante genetische Material enthalten, und die Verwendung solcher Mikroorganismen in einem biotechnologischen Verfahren zur Herstellung von Biotin.

Biotin (Vitamin H) ist ein für Mensch und Tier wichtiges Vitamin, dessen Mangel beispielsweise Seborrhoe, Dermatitis, Appetitlosigkeit und Müdigkeit hervorrufen kann. Dementsprechend ist Biotin ein nützlicher Zusatzstoff für Lebens- und Futtermittel.

Die Herstellung von Biotin mit Methoden der synthetischen organischen Chemie ist aufwendig und kostspielig. Aus diesem Grunde finden biotechnologische Verfahren, in denen Biotin mit Hilfe von Mikroorganismen aus billigen Ausgangsmaterialien wie Glucose synthetisiert werden kann, zunehmend Beachtung.

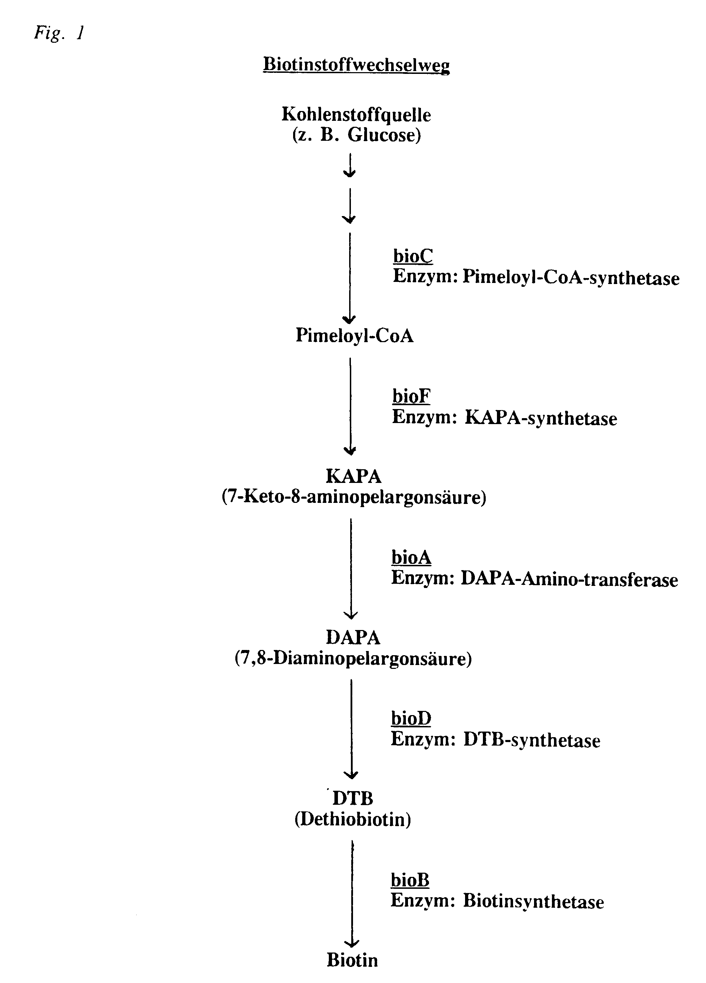

Escherichia coli (E. coli) ist ein Mikroorganismus, der in der Lage ist, ausgehend von einfachen Kohlenstoffquellen wie Glycerin oder Glucose Biotin zu synthetisieren (Fig. 1). Die Gene, die für die Biosynthese von Biotin in E. coli verantwortlich sind, liegen in einem Operon vor, das bereits kloniert wurde und die fünf Gene bioA, bioB bioC, bioD und bioF (im folgenden auch als bio-Gene bezeichnet) umfaßt (Gupta et al., Gene 1:331-345; 1977). Diese Gene werden von einer Promotor-Operator-Region, die sich zwischen den Genen bioA und bioB befindet, in zwei verschiedene Richtungen transkribiert. Bezüglich der konventionellen Genkarte liegen die Gene bioB, bioF, bioC und bioD rechts und das Gen bioA links von der Promotor-Operator-Region. Die DNA links der Promotor-Operator-Region umfaßt stromabwärts des bioA-Gens ein weiteres Gen mit der Bezeichnung ORFI (ORF = open reading frame), das für ein Polypeptid mit 158 Aminosäuren codiert und zusammen mit dem bioA-Gen transkribiert wird (Otsuka et al., J. Biol. Chem., 263:19577-19585; 1988). Die Funktion dieses Gens ist bislang unbekannt. Andere Stämme aus der Familie der Enterobakterien, beispielsweise der Gattung Salmonella oder Citrobacter, weisen einen zu E. coli analogen Aufbau des Biotin-Operons auf (Shiu-an und Campbell, Gene 67: 203-211; 1988).

Es sind bereits biotechnologische Verfahren zur Herstellung von Biotin bekannt, die mittels Mikroorganismen durchgeführt werden, die mit dem klonierten Biotin-Operon von E. coli transformiert sind. Diese Verfahren werden ausgehend von Glucose durchgeführt. Die EP-B-236 429 beschreibt beispielsweise Mikroorganismen, die mit dem Biotin-Operon von E. coli transformiert sind, wobei die Wirtsorganismen in ihrem birA/bioR-Gen mutiert sind.

In der EP-A-316 229 werden E. coli-Mutanten beschrieben, die weniger Acetat produzieren und ebenfalls mit dem klonierten Biotin-Operon transformiert wurden.

Die EP-A-449 724 offenbart mit dem Biotin-Operon transformierte Mikroorganismen, die zusätzlich Mutationen aufweisen, die einen geringeren Glucoseverbrauch zur Folge haben.

Aus der EP-A-266 240 ist ferner die Klonierung der für die Biotin-Synthese in Bacillus sphaericus verantwortlichen Gene und ein darauf aufbauendes Verfahren zur Herstellung von Biotin bekannt. Bedingt durch den Metabolismus von Bacillus sphaericus muß dieses Verfahren ausgehend von kostspieliger Pimelinsäure durchgeführt werden.

Die Ausbeuten, die nach den bislang bekannten biotechnologischen Verfahren erhalten werden, sind jedoch unter wirtschaftlichen Gesichtspunkten bislang nicht zufriedenstellend.

Aufgabe der vorliegenden Erfindung ist es daher, ein biotechnologisches Verfahren zur Herstellung von Biotin bereitzustellen, das höhere Ausbeuten an Biotin ermöglicht und damit wirtschaftlicher ist.

Diese Aufgabe wurde unter Verwendung von DNA-Fragmenten und Vektoren gelöst, die die Gene bioB, bioF, bioC, bioD und bioA oder deren funktionell äquivalente genetische Varianten und Mutanten aus Enterobakterien umfassen, wobei diese Gene in einer Transkriptionseinheit organisiert sind.

Unter Transkriptionseinheit wird hierbei eine DNA-Sequenz verstanden, in der die Gene in einer Transkriptionsrichtung angeordnet sind und unter gemeinsamer Transkriptionskontrolle in ein durchgehendes Transkript überschrieben werden, wobei die DNA-Sequenz neben den jeweiligen Genen auch die für die Genexpression erforderlichen genetischen Kontrollelemente wie Promotoren und Ribosomenbindungsstellen umfaßt.

Unter "funktionell äquivalenten genetischen Varianten und Mutanten" werden Gene verstanden, die sich von den Wildtyp-Genen der Ursprungsorganismen, d. h. der Enterobakterien, ableiten und Basenaustausche im Rahmen der bekannten Degeneration des genetischen Codes aufweisen. Derartige Basenaustausche können natürlichen Ursprungs oder künstlich erzeugt sein, beispielsweise um die Gensequenz an die bevorzugte Codon-Verwendung eines bestimmten Mikroorganismus, in dem eine Expression erfolgen soll, anzupassen. Die genetischen Varianten und Mutanten umfassen ferner Deletionen, Insertionen und Substitutionen von Basen oder Codons, die das Genprodukt einer derart veränderten Sequenz in seiner Funktion grundsätzlich intakt lassen. Umfaßt sind insbesondere Sequenzen, die unter den üblichen Hybridisierungsbedingungen, d.h. bei Temperaturen zwischen 55 und 66°C und bei 0,03 bis 0,3M Salzanteil, mit den Wildtyp-Sequenzen hybridisieren, also Sequenzen, die zu den Wildtyp-Sequenzen eine hohe Homologie, beispielsweise höher als 70%, aufweisen.

Fig. 1 zeigt die Enzyme des Stoffwechselwegs der Biotin-Biosynthese.

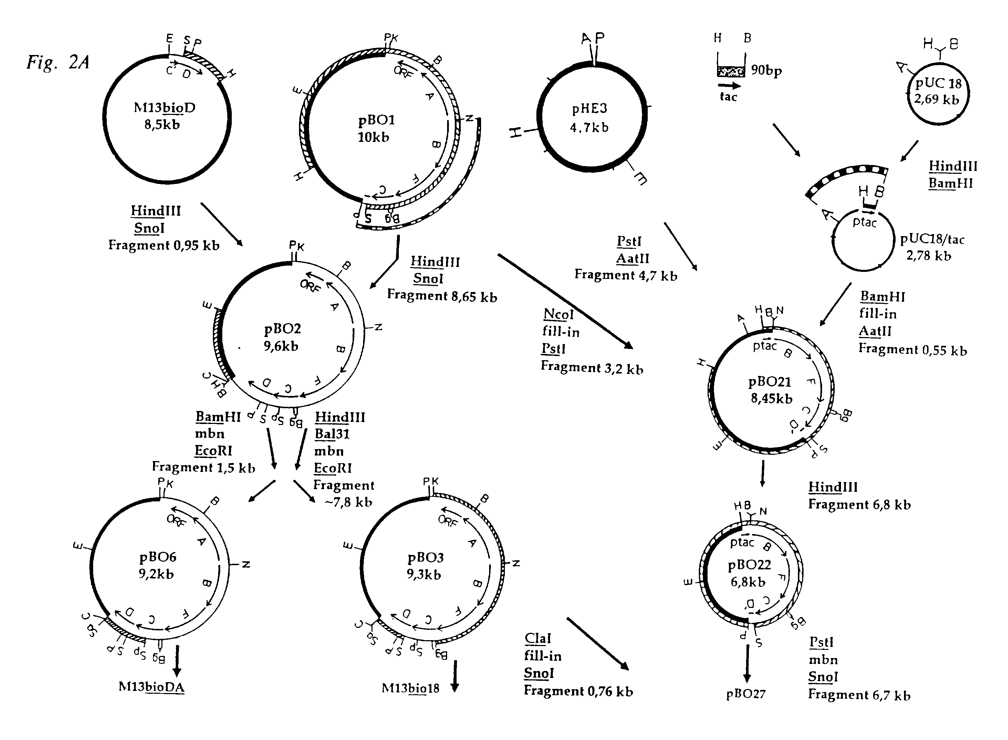

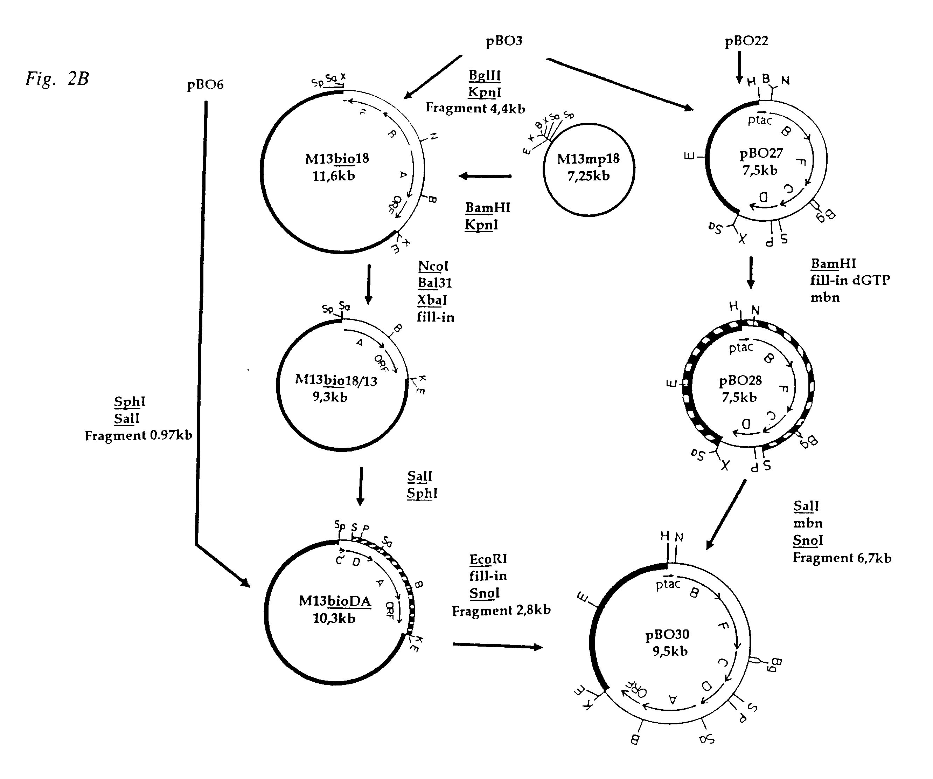

Fig. 2 zeigt das Konstruktionsschema für das Plasmid pB030.

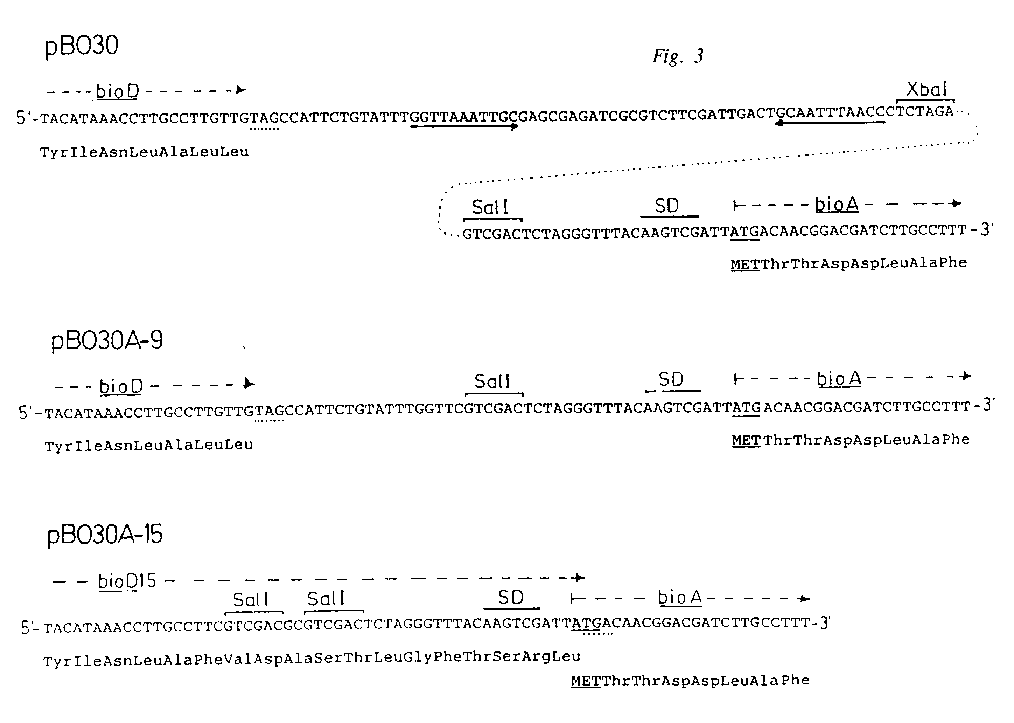

Fig. 3 zeigt die DNA-Sequenz der Plasmide pB030, pB030A-9 und pB030A-15 für den Bereich des 3'-Endes des bioD-Gens und des 5'-Endes des bioA-Gens (durchbrochene Pfeile; das bioA-Start-codon ist unterstrichen, das bioD-Stopcodon punktiert dargestellt) zusammen mit den für die Plasmidkonstruktion relevanten Restriktionsschnittstellen und der Shine-Dalgarno (SD)-Sequenz des bioA-Gens. Potentielle "stem-loop"-Strukturen sind mit durchgehenden Pfeilen gekennzeichnet.

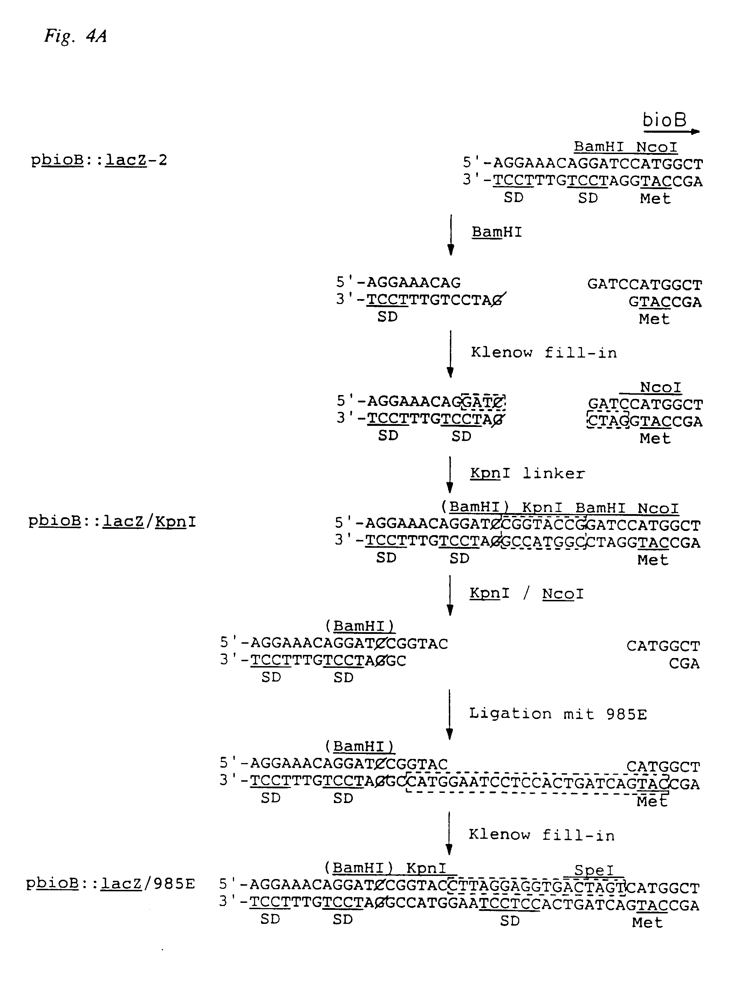

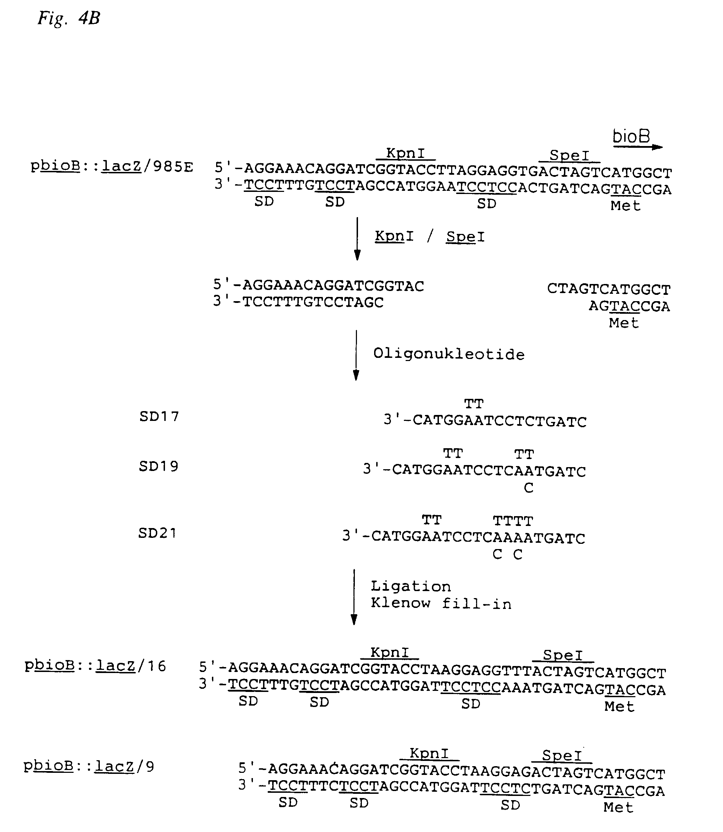

Fig. 4 zeigt die Schritte zur Variation der Sequenz stromaufwärts des bioB-Gens ausgehend von Plasmid pbioB::lacZ-2 zur Konstruktion verbesserter Ribosomenbindungsstellen unter Angabe der benutzten Restriktionsschnittstellen, der jeweiligen Shine-Dalgarno-Sequenzen (SD) und des bioB-Startcodons (Met). Dargestellt sind die Sequenzen stromaufwärts des bioB-Gens und der 5'-Terminus des bioB-Gens. Die gestrichelten Linien kennzeichnen das inserierte Oligonukleotid 985E. Durchgestrichene Nukleotide sollten nach der Theorie vorhanden sein, fehlen aber in Plasmid pbioB::lacZ/985E und den davon abgeleiteten Plasmiden pbioB::lacZ/9 und pbioB::lacZ/16, was den Verlust einer BamHI-Stelle (BamHI) zur Folge hat. "fill-in": Auffüllen mit Klenow-Polymerase.

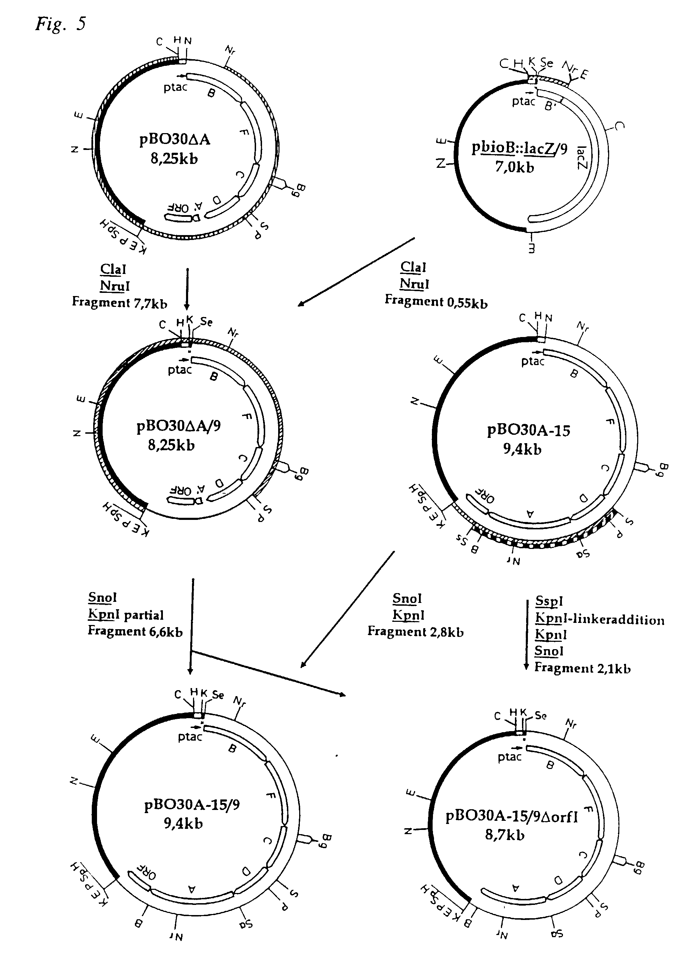

Fig. 5 zeigt das Konstruktionsschema für die Plasmide pBO30A-15/9 und pBO30A-15/9ΔorfI.

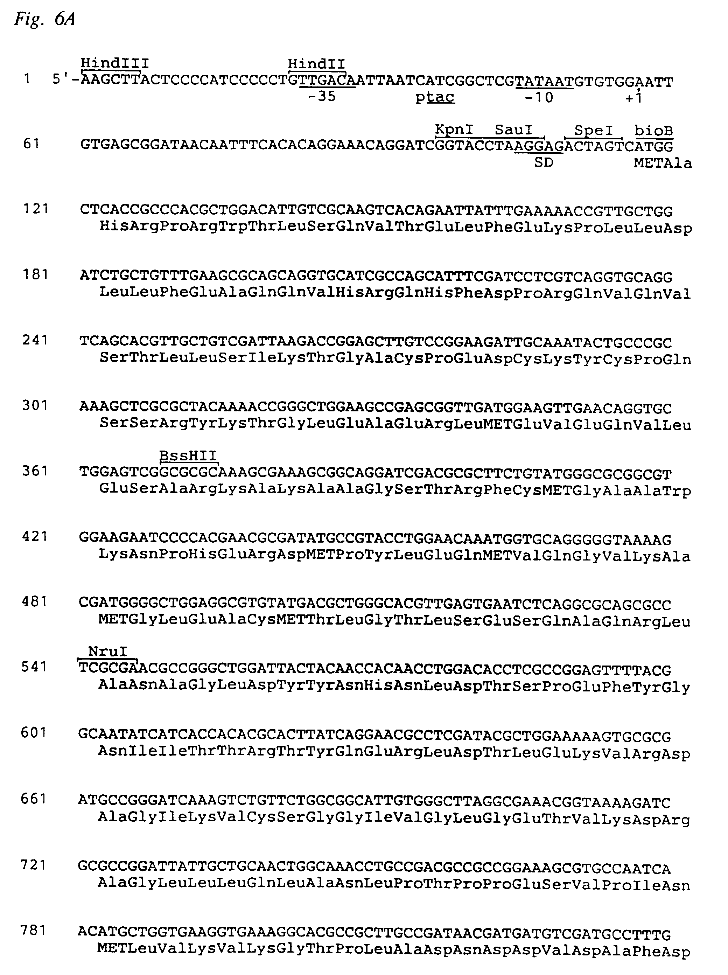

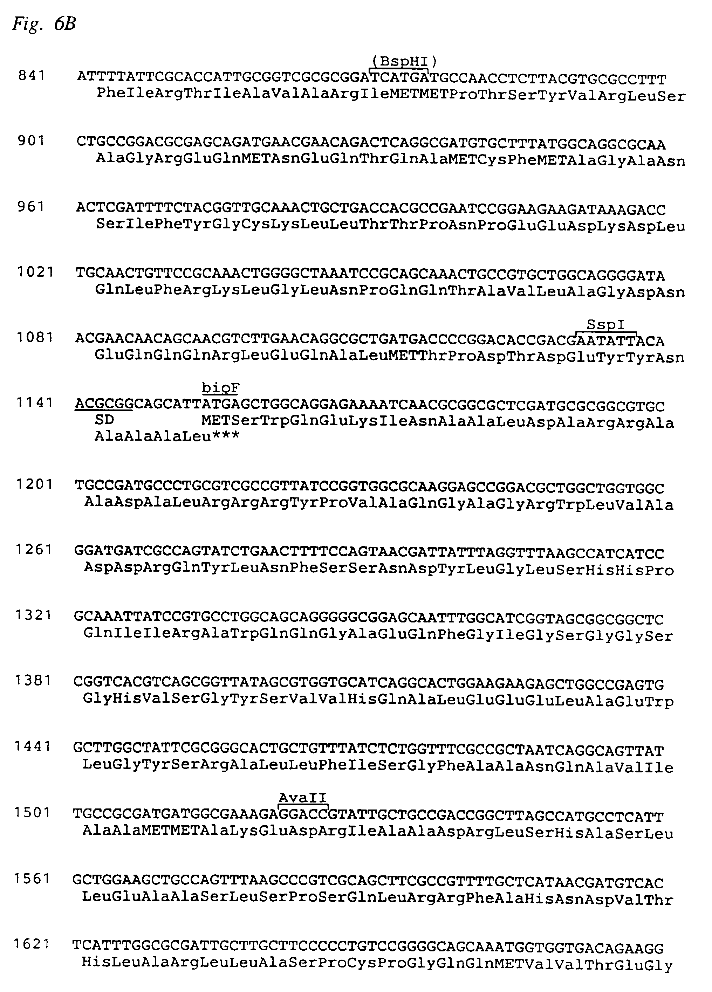

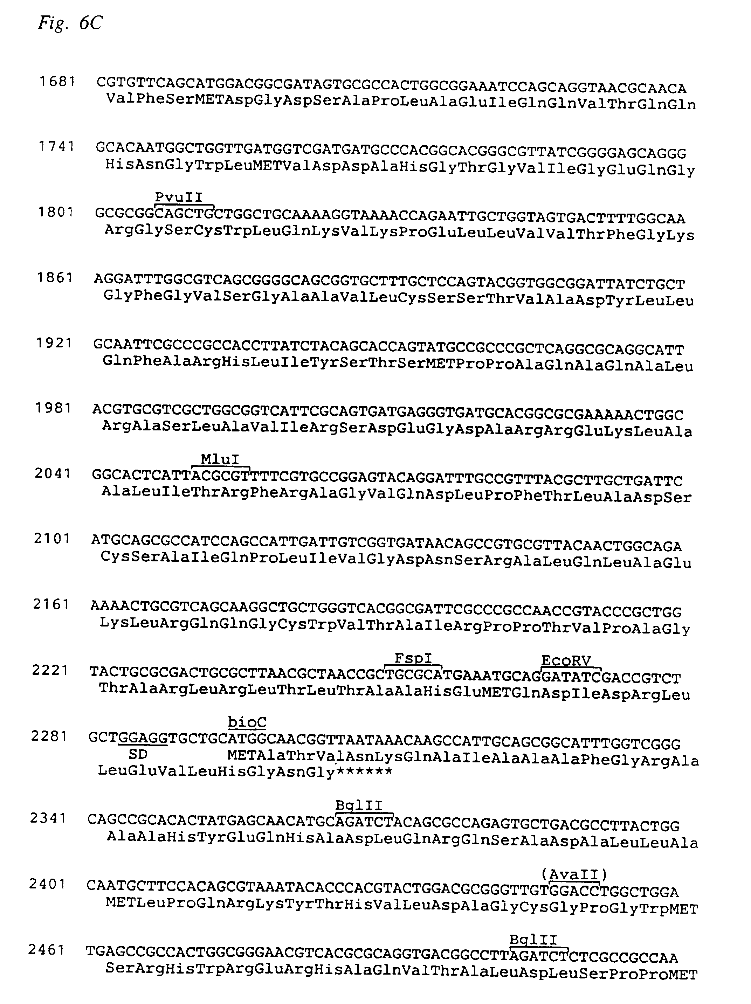

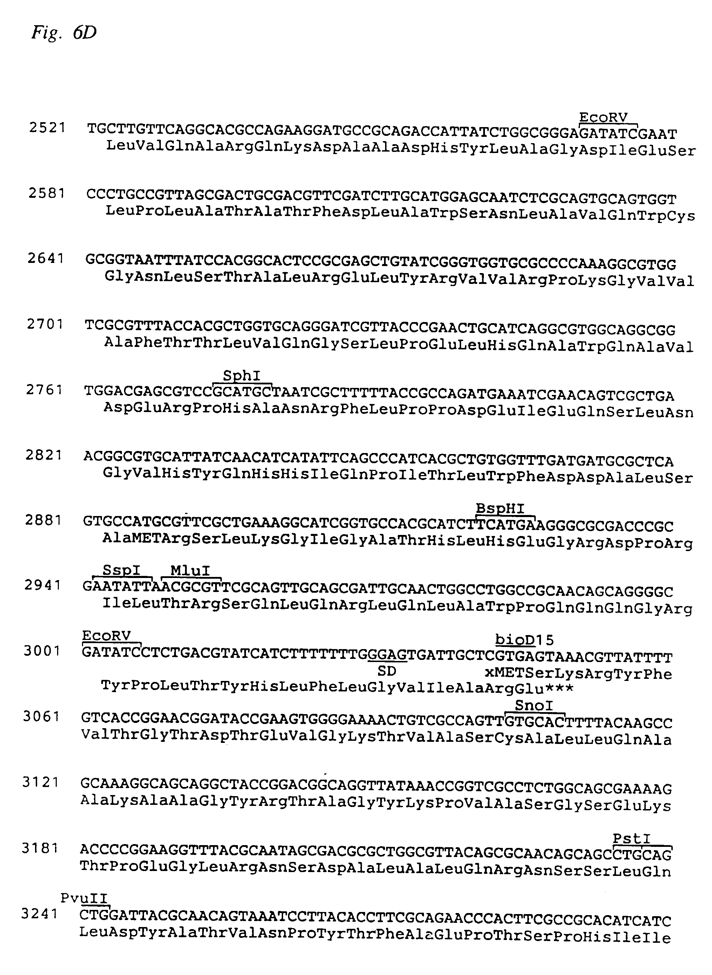

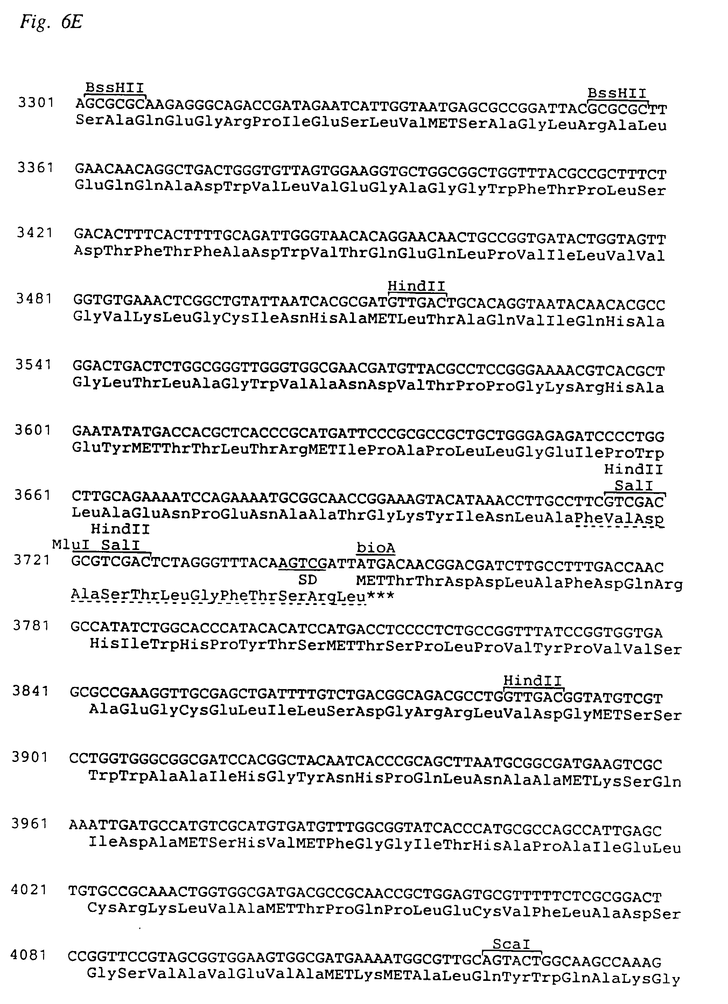

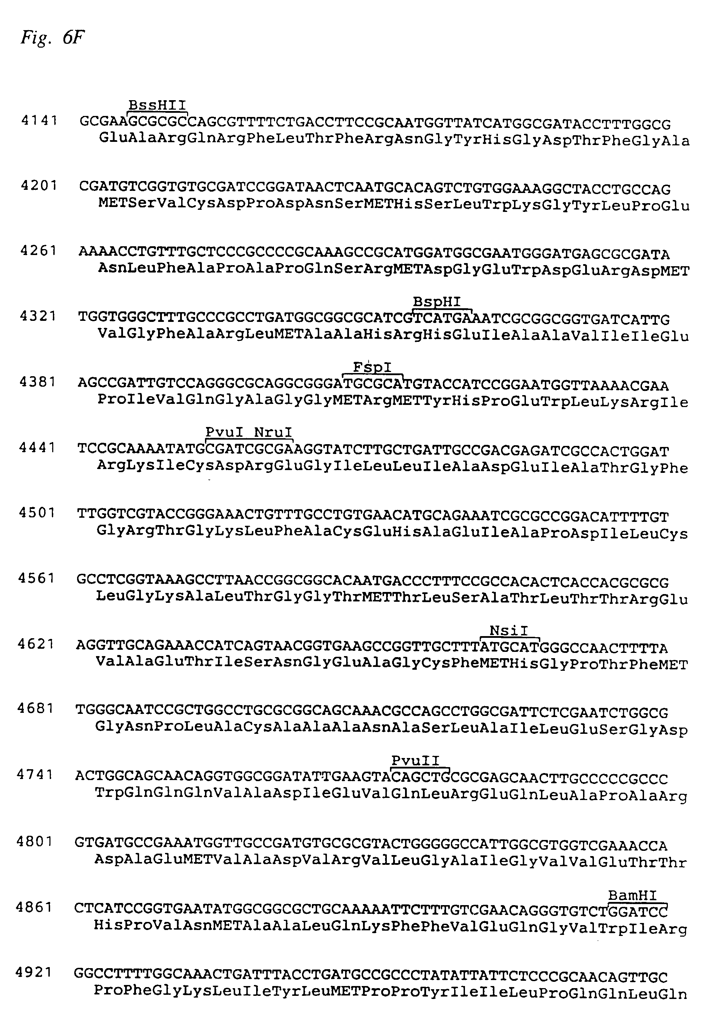

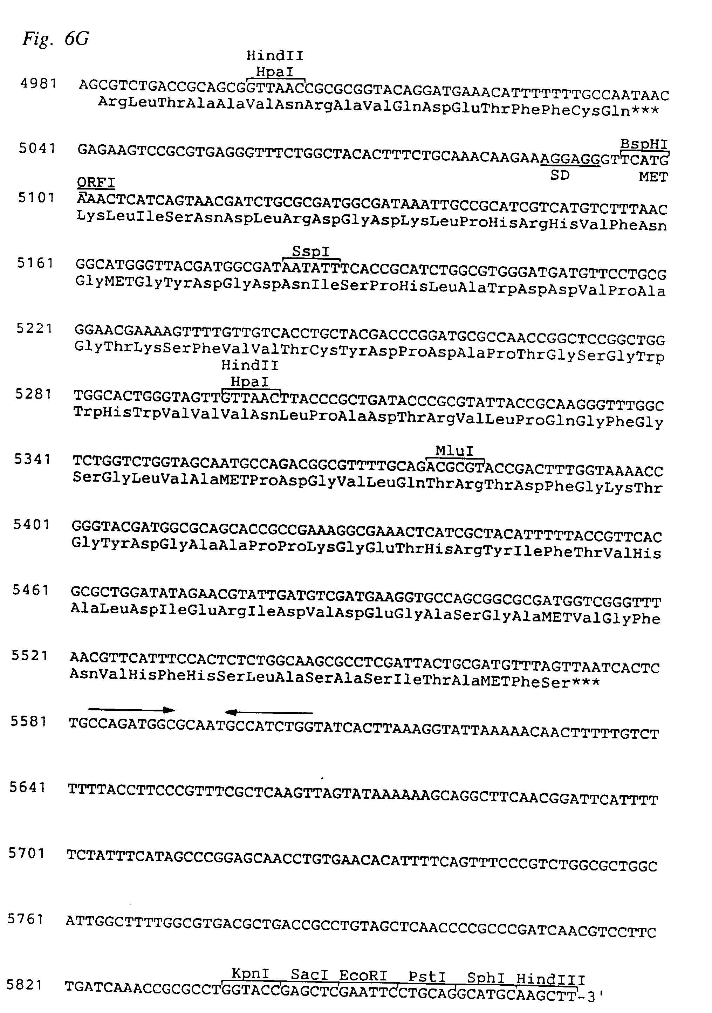

Fig. 6 zeigt die DNA- und Aminosäuresequenz der in Plasmid pBO30A-15/9 für die Biotin-Biosynthese codierenden Gene zusammen mit den genetischen Kontrollelementen (SD: Shine-Dalgarno-Sequenz). Kursiv dargestellte Aminosäuren am COOH-Terminus des bioD15-Gens stellen Substitutionen gegenüber der Wildtyp-Sequenz des bioD-Gens von E. coli dar.

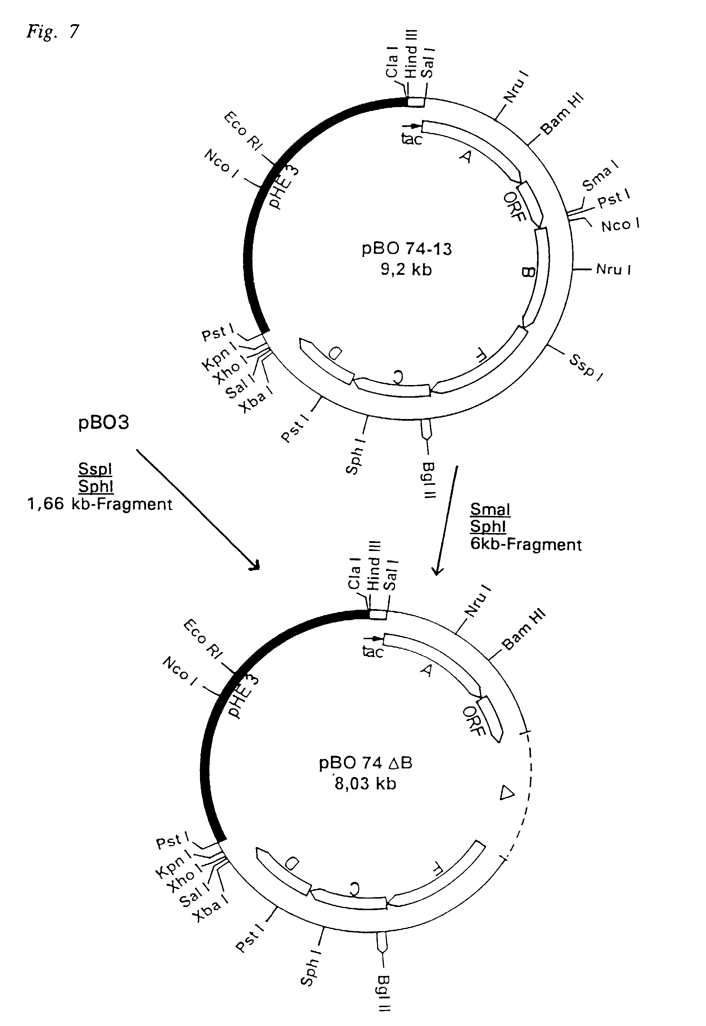

Fig. 7 zeigt das Konstruktionsschema für Plasmid pBO74ΔB ausgehend von den Plasmiden pBO74-13 und pBO3; Pfeile geben die Lage und die Orientierung des tac-Promotors und der bio-Gene an. Der Vektoranteil der Plasmide ist fett dargestellt. Durchbrochene Linien geben den Umfang der Deletion des bioB-Gens wieder.

In den Fig. 2 und 5 bedeuten A: AatII; B: BamHI; Bg: BgIII; C: ClaI;E: EcoRI; H: HindIII; K: KpnI; N: NcoI; Nr: Nrul;P: PstI; S: SnoI; Sa: SalI; Se: SseI; Sp: SphI; Ss: SspI; und X: XbaI. "fill-in": Auffüllen rezessiver 3'-Enden mit Klenow-Polymerase; mbn: Entfernen überhängender 5'- oder 3'-Enden mit "Mung Bean Nuclease; Bal31: progressive Deletion von DNA mit Exonuklease Bal31. Der Vektoranteil der Plasmide ist fett dargestellt. Die unterschiedlich schraffierten Teile in den Plasmiden wurden jeweils für den nachfolgenden Klonierungsschritt verwendet. Pfeile geben die Lage und die Orientierung der bio-Gene an.

Zur Konstruktion der erfindungsgemäßen DNA-Fragmente und Vektoren werden die Gene des Biotin-Operons zunächst zweckmäßig aus dem Chromosom eines geeigneten Mikroorganismus isoliert und anschließend unter der Kontrolle genregulatorischer Elemente wie Promotoren und Ribosomenbindungsstellen so miteinander verknüpft, daß sie organisiert in einer einzigen Transkriptionseinheit vorliegen. Als Ausgangsmaterial für die Isolierung der bio-Gene können Bakterienstämme aus der Familie der Enterobakterien dienen, beispielsweise der Gattung Escherichia, Salmonella oder Citrobacter. Zweckmäßig ist das Ausgangsmaterial ein Mikroorganismus der Spezies Escherichia coli, die am besten charakterisiert ist.

Die Konstruktion der erfindungsgemäßen DNA-Fragmente und Vektoren kann beispielsweise ausgehend von einer Genbank eines geeigneten Mikroorganismus wie E. coli erfolgen, aus der die bio-Gene oder Fragmente davon durch Hybridisierung mit markierten Oligonukleotiden, die Teilsequenzen der bio-Gene enthalten, in bekannter Weise isoliert und kloniert werden können. Anschließend werden die isolierten und klonierten bio-Gene mit den bekannten Methoden der DNA-Rekombination unter der Kontrolle eines gemeinsamen Promotors so miteinander verknüpft, daß sie als eine einzige Transkriptionseinheit vorliegen. Zweckmäßig erfolgt die Anordnung der bio-Gene derart, daß das bioA-Gen stromabwärts der Gene bioB, bioF, bioC und bioD liegt, die im Wildtyp-Operon von E. coli bereits in einer Transkriptionseinheit vorliegen. Das bioB-Gen kodiert mit der Biotinsynthase das Schlüsselenzym des gesamten Biotin-Synthesewegs, da die Umsetzung von Dethiobiotin zu Biotin durch Biotinsynthase bislang den geschwindigkeitsbestimmenden Schritt des fünfstufigen Biotin-Synthesewegs darstellt. Das bioB-Gen ist daher zweckmäßig das erste Gen innerhalb der Transkriptionseinheit, da dann wegen der Nähe zum Promotor eine optimale Expression dieses Gens erfolgen kann (Fig. 2, 4, 5 und 6).

Die zweite Transkriptionseinheit im Wildtyp-Biotin-Operon von E. coli, die das bioA-Gen enthält, umfaßt außerdem ein weiteres Gen, ORFI, das für ein Polypeptid mit 158 Aminosäuren kodiert. Versuche, die mit Expressionsplasmiden durchgeführt wurden, in denen kein ORFI-Gen anwesend ist, zeigen, daß dieses Gen unter den üblichen Fermentationsbedingungen für die Biotin-Biosynthese nicht essentiell ist. Es ist allerdings nicht auszuschließen, daß diesem in seiner Funktion bislang unbekannten Polypeptid unter bestimmten Bedingungen auch eine Rolle bei der Biotin-Synthese zukommt. Obwohl die Anwesenheit des ORFI-Gens in den erfindungsgemäßen DNA-Fragmenten also nicht zwingend notwendig ist, umfaßt die Transkriptionseinheit mit den bio-Genen in einer zweckmäßigen Ausführungsform zusätzlich auch das ORFI-Gen. (Fig. 2, 5 und 6).

In den erfindungsgemäßen DNA-Fragmenten und Vektoren stehen die bio-Gene vorteilhaft nicht unter der Kontrolle des natürlichen Biotin-Promotors von E. coli. Vielmehr werden die bio-Gene zur Verbesserung der Transkription zweckmäßig unter die Kontrolle eines starken Fremdpromotors gestellt. Die Wahl des Promotors hängt von den gewünschten Expressionsbedingungen ab, beispielsweise davon, ob eine konstitutive oder induzierte Expression gewünscht wird, oder von dem Mikroorganismus, in dem die Expression erfolgen soll. Geeignete Promotoren sind beispielsweise die Promotoren P_{L} und P_{R} des Phagen Lambda (vgl. Schauder et al., Gene 52:279-283; 1987), der Promotor pxylS des TOL-Plasmids von Pseudomonas putida mit dem benachbarten Regulatorgen xyl R (Franklin et al., J. Bacteriol. 154: 676-685; 1983), der trc-Promotor (Amann et al., Gene 69:301-315; 1988), der trp-Promotor (Amann et al., Gene 25:167-178; 1983), der Promotor pdegQ aus Bacillus subtilis, der in der Stationärphase aktiv ist (Dahl et al., J. Bacteriol. 173:1539-1547; 1991) und der lacUV5-Promotor (Amann et al., Gene 25:167-178; 1983). Bevorzugt wird als Promotor der tac-Promotor gewählt, ein Hybrid aus dem trp- und dem lacUV5-Promotor von E. coli, der als konstitutiver oder induzierbarer Promotor eingesetzt werden kann (Russell und Bennett, Gene 20:231-243; 1982).

Es wurde außerdem gefunden, daß die Expression des bioA-Gens in der oben beschriebenen bevorzugten Anordnung weiter verbessert werden kann, wenn der Abstand zwischen den in der Transkriptionseinheit aufeinanderfolgenden Genen bioD und bioA möglichst kurz ist, d. h. vorzugsweise weniger als 50bp (Basenpaare) beträgt. Überraschend wurde festgestellt, daß die Expression besonders hoch ist, wenn die Sequenz des 3'-Endes des bioD-Gens, die für den COOH-Terminus der Dethiobiotin (DTB)-Synthetase kodiert, gleichzeitig die Ribosomenbindungsstelle des nachfolgenden bioA-Gens beinhaltet. Vorteilhaft liegt gleichzeitig eine Überlappung der Leseraster der Gene bioD und bioA vor. Eine derartige Konstellation läßt sich erreichen, wenn man das 5'-Ende des bioA-Gens zusammen mit dessen Ribosomenbindungsstelle so mit dem bioD-Gen fusioniert, daß dessen 3'-Ende durch die Sequenz mit der Ribosomenbindungsstelle stromaufwärts des bioA-Gens und, gegebenenfalls, den 5'-Terminus des bioA-Gens substituiert wird (Fig. 3 und 6; Seq ID No: 1, 6 und 8-16). Dieser Effekt ist umso überraschender, als bei einer derartigen Fusion der COOH-Terminus der DTB-Synthetase ausgetauscht werden kann, ohne daß das Enzym seine Aktivität verliert. Ähnliche Überlappungen finden sich auch im Wildtyp-Biotin-Operon von E. coli zwischen den Leserastern der Gene bioB, bioF, bioC und bioD.

Die Expression des bioB-Gens läßt sich durch Optimierung der Ribosomenbindungsstelle vor dem bioB-Gen weiter optimieren. Zweckmäßig geht man hierbei von einem Konstrukt aus, in dem das bioB-Gen bereits unter der Kontrolle eines starken Promotors, z.B. des tac-Promotors, steht. Die Optimierung der Ribosomenbindungsstelle des bioB-Gens, d.h. die Variation der Shine-Dalgarno-Sequenz und deren Abstand zum 5'-Ende des Strukturgens, kann mittels der üblichen Methoden der DNA-Rekombination erfolgen. Der Einfluß einer bestimmten Ribosomenbindungsstelle auf die Translation kann in an sich bekannter Weise, beispielsweise durch Genfusion des zu testenden Gens mit dem lacZ-Gen und anschließendem Test mit dem chromogenen Substrat 5-Brom-4-chlor-3-indolyl-β-D-galactopyranosid (X-Gal), bestimmt werden.

DNA-Fragmente, die die bio-Gene in einer Transkriptionseinheit umfassen, können mit Hilfe der bekannten Techniken der DNA-Rekombination in eine Vielzahl von Vektoren eingebaut werden. Auf diese Weise wurden beispielsweise die Plasmide pBO30A-15/9 (Fig. 5 und 6, Seq ID No: 1 und 6; Beispiel 1.5.2) und pBO47 (Beispiel 1.7) erhalten. Plasmid pBO30A-15/9 wurde am 28. 9. 1992 bei der Deutschen Sammlung für Mikroorganismen und Zellkulturen GmbH, D-3300 Braunschweig, Mascheroderweg lb, in E. coli XL1-Blue und E. coli BM4062 unter den Hinterlegungsnummern DSM 7246 bzw. 7247, und am 17. 9. 1993 in E. coli ED8767 unter der Hinterlegungsnummer DSM 8554 hinterlegt. Plasmid pBO47 wurde am 17. 9.1993 bei der Deutschen Sammlung für Mikroorganismen und Zellkulturen GmbH in Agrobacterium/ Rhizobium sp HK4 unter der Hinterlegungsnummer DSM 8555 hinterlegt.

Abhängig von der Art der gewählten Vektoren können die Gene für die Enzyme des Biotin-Synthesewegs in verschiedenen Organismen exprimiert werden. Als Vektoren eignen sich sowohl Vektoren mit spezifischem Wirtsspektrum als auch Vektoren mit breitem Wirtsspektrum ("broad host range"). Beispiele für Vektoren mit spezifischem Wirtsspektrum, z.B. für E. coli, sind pBR322 (Bolivar et al., Gene 2:95-113; 1977), pUC18/19 (Yanisch-Perron et al., Gene 33:103-119; 1985), pK18/19 (Pridmore, Gene 56:309-312; 1987) und pRA95 (erhältlich von Nycomed Pharma AS, Hvidovre, Dänemark).

Als "broad host range" Vektoren können alle Vektoren eingesetzt werden, die für Gram-negative Bakterien geeignet sind. Beispiele für solche "broad host range" Vektoren sind pRK290 (Ditta et al., Proc. Natl. Acad. Sci. USA 77:7347-7351; 1980), pKT240 (Bagdasarian et al., Gene 26:273-282; 1983), Derivate von pRK290 wie pLAFR1 (Long et al., Nature 298:485-488; 1982) und pRK290X (Alvarez-Morales et al., Nucl. Acid. Res. 14:4207-4227; 1986), Derivate von pKT240 wie pMMB66EH (Fürste et al., Gene 48:119-131; 1986) oder pGSS33 (Sharpe, Gene 29:93-102; 1984).

Zur Herstellung der Produktionsstämme für die Fermentation, d. h. der Stämme für die Biotin-Produktion, muß das erfindungsgemäße DNA-Fragment in die gewünschten und zur Expression geeigneten Wirtsstämme eingebracht werden. Mikroorganismen, die zur Expression der bio-Gene geeignet sind, vorzugsweise Stämme mit weitem Substratspektrum, sind beispielsweise Enterobakterien, vorzugsweise der Gattung Escherichia, oder Mikroorganismen der Gattung Rhizobium, Agrobacterium,RhizobiumAgrobacterium, Acinetobacter, Azotobacter, Pseudomonas und Comamonas. Besonders bevorzugt sind Mikroorganismen der Spezies E. coli, Rhizobium-/Agrobacterium sp. HK4 (wie beschrieben in der EP-B 158 194), Pseudomonas mendocina, Pseudomonas aeruginosa oder Acinetobacter calcoaceticus. Die Mikroorganismen können das erfindungsgemäße DNA-Fragment entweder auf einem Vektormolekül oder integriert in ihrem Chromosom enthalten. Das Einbringen des DNA-Fragments in die Mikroorganismen kann beispielsweise durch Transformation oder Konjugation erfolgen. Zweckmäßig werden die ausgewählten Mikroorganismen in an sich bekannter Weise mit Vektoren transformiert, die die erfindungsgemäßen DNA-Fragmente enthalten. Geeignete Produktionsstämme sind beispielsweise E. coli XL1-Blue, E. coli BM4062 und E. coli ED8767, jeweils enthaltend Plasmid pBO30A-15/9 (DSM 7246, DSM 7247 und DSM 8554) und Aqrobacterium/ Rhizobium sp HK4 mit Plasmid pB047 (DSM 8555).

Die Isolierung der transformierten Wirtsstämme erfolgt zweckmäßig aus einem selektiven Nährmedium, dem ein Antibiotikum zugesetzt wird, gegen das die Wirtsstämme durch ein auf dem Vektor oder DNA-Fragment befindliches Markergen resistent sind.

Die biotechnologische Herstellung von Biotin erfolgt unter Verwendung der Mikroorganismen, die die erfindungsgemäßen DNA-Fragmente oder Vektoren enthalten. Das Verfahren zur Herstellung von Biotin erfolgt in üblicher Weise in Kulturen ausgehend von einer als Wachstumssubstrat für den jeweiligen Mikroorganismus geeigneten Kohlenstoffquelle, die schließlich in Biotin umgewandelt wird. Als Kohlenstoffquelle geeignet sind insbesondere einfache Zuckermoleküle, beispielsweise Glucose, oder Glycerin. Dementsprechend können als Wachstumsmedien handelsübliche Medien wie beispielsweise Nutrient Yeast Broth (NYB: Nutrient Broth No. 2, Oxoid, 25g/l; Hefe-Extrakt, Oxoid, 5g/l) oder Glycerin-und Glucose-Minimalmedien verwendet werden.

Bevorzugt erfolgt die Fermentation, d.h., die Herstellung von Biotin, als sog. "fed-batch-Verfahren", d.h., in einer Batch-Fermentation, der kontinuierlich oder in Intervallen ein Volumenstrom mit frischen Nährstoffen zugeführt wird, wobei jedoch keine Kulturlösung abgezogen wird. In einem solchen Verfahren wird als "feed" vorzugsweise eine Glycerinlösung mit variabler, der jeweiligen Biomasseentwicklung angepaßter Zuflußrate zugeführt.

Die Fermentation erfolgt innerhalb der für die jeweiligen Mikroorganismen physiologisch verträglichen pH- und Temperaturbereiche. Zweckmäßig liegt der pH-Wert innerhalb eines Bereichs von 6 bis 8 und die Temperatur innerhalb eines Bereichs von 20 bis 45°C.

Durch Variation der Nährstoffe im Medium und durch Anpassung der Fermentationsbedingungen an den jeweiligen Mikroorganismus in üblicher Weise kann die Ausbeute an Biotin weiter verbessert werden.

Die vorliegende Erfindung wird in den nachfolgenden Beispielen weiter erläutert.

### Allgemeine Verfahren:

Restriktionsendonukleasen wurden mit 3 bis 5 Units/µg DNA nach den Angaben der Hersteller eingesetzt. Markierung und Phosphorylierung von DNA-Linkern (bezogen von Boehringer Mannheim, BRD) zum Einbau von Restriktionsschnittstellen, und von synthetischen Oligonukleotiden (bezogen von Microsynth, Windisch, CH), beispielsweise zur Verwendung als Sonden für DNA/DNA-Hybridisierungen und als "Primer" für Sequenzierungsreaktionen, erfolgten mit T4-Polynukleotid-Kinase (Boehringer Mannheim, BRD) nach Sambrook et al. (Molecular Cloning: A laboratory manual. 2nd edition, Cold Spring Harbour Laboratory, Cold Spring Harbour, NY; 11.31 und 5.68; 1989). Ligationsreaktionen erfolgten mit T4-DNA-Ligase nach den Angaben der Hersteller.

DNA-Sequenzierungen erfolgten nach der Kettenabbruchsmethode nach Sanger et al. (Proc. Natl. Acad. Sci. USA 94:5463-5467; 1977). Alle Sequenzreaktionen wurden mit dem Sequenase-Kit von United States Biochemicals (Cleveland, OH, USA) nach dem Protokoll des Herstellers durchgeführt. Sequenase (Version 2.0, eine gentechnisch veränderte T7-DNA-Polymerase) ergab gleichmäßige, gut lesbare DNA-Sequenzen über mehr als 600bp; Kompressionen in GC-reichen DNA-Bereichen konnten leicht aufgelöst werden, wenn anstatt dGTP das Nukleotid dITP verwendet wurde. Als Matrizen für die Sequenzreaktion wurden in der Regel die Einzelstrang-Formen der Vektoren M13mp18/19 (Yanisch-Perron et al., 1985, ibid.) oder pBluescript KS⁺/SK⁺ (ap^{R} lacZ'; erhältlich von Stratagene, La Jolla, CA) verwendet, die nach Messing (Methods Enzymol. 101:20-79; 1983) isoliert wurden. Zur Sequenzierung doppelsträngiger Plasmid-DNA wurde die Plasmid-DNA mittels CsCl -Gradienten oder "Gene Clean" (BIO 101, La Jolla, CA) gereingt. Als radioaktiv markiertes Nukleotid wurde α[³⁵S]-dATP (NEN-Du Pont, NEG-034H) verwendet. Die elektrophoretische Auftrennung erfolgte entweder über die üblichen 4% bzw. 6% Bis/Acrylamid-Gele mit 7M Harnstoff und lx TBE-Puffer (90 mM Tris, 90 mM Borsäure, 2.5 mM EDTA), oder aber über Gele aus 5% HydroLink Long Ranger (AT Biochem, Malvern, PA, USA, via Chemie Brunschwig, Basel) mit 7M Harnstoff und 1,2 x TBE-Puffer. Die Gele waren 550 mm lang und 0,2 mm dick; die Elektrophorese erfolgte in einer LKB Macrophor-Apparatur mit Thermostat bei einer Spannung von 2100V und einer Temperatur von 60°C. Anschließend wurden die Gele auf Whatman 3 MM Papier getrocknet und mit Röntgenfilm Fuji RX oder Amersham Hyperfilm ßmax autoradiografiert.

Die Isolierung extrachromosomaler DNA erfolgte entweder in kleineren Mengen nach der "rapid alkaline SDS" ("Miniprep")-Methode nach Birnboim und Doly (Nucl. Acid. Res. 7:1513-1523; 1979), oder, zur Isolierung größerer Mengen, durch Cäsiumchlorid-Dichtegradienten-Zentrifugation nach einer modifizierten Methode nach Clewell und Helsinki (Proc. Natl. Acad. Sci. USA 42:1159-1166; 1969). Alternativ wurden QIAGEN-packs der Fa. DIAGEN, Düsseldorf (BRD) verwendet.

Zur Transformation von E. coli mit Plasmid-DNA wurden die Zellen nach Cohen et al. (Proc. Natl. Acad. Sci. USA 69:2110-2114; 1972) in 50mM CaCl₂ kompetent gemacht. Die Transformation mit Plasmid-DNA und die Selektion plasmidtragender Klone erfolgte nach Sambrook et al. (1989; ibid. 1.82-1.84).

### BEISPIEL 1

### Klonierung des E. coli Biotin-Operons in einer einzigen Transkriptionseinheit

### 1.1 Konstruktion von pBO1 und M13bioD

Zur Klonierung der bio-Gene wurde die chromosomale DNA von E. coli DSM 498 (K12 "Wildtyp"; Deutsche Sammlung für Mikroorganismen und Zellkulturen GmbH) isoliert. Die Isolierung erfolgte im wesentlichen nach Hahn und Hennecke (Mol. Gen. Genet. 193:46-52; 1984). Anschließend wurden 2 µg Gesamt-DNA von E. coli DSM 498 mit dem Restriktionsenzym PstI geschnitten. Die DNA-Fragmente wurden in einem horizontalen 0,7% Agarosegel in üblicher Weise (Sambrook et al., 1989, ibid.; 6.19 bis 6.9) elektrophoretisch aufgetrennt und auf "Gene Screen" Membranen (Nylonmembranen von NEN-Du Pont) transferiert (Southern, J. Mol. Biol., 98:503-517; 1975). Die DNA wurde durch zweistündige Inkubation bei 80°C im Vakuumofen auf den getrockneten Filtern fixiert. Zur Identifizierung von DNA-Fragmenten mit dem bio-Operon wurde ein 25 Nukleotide langes synthetisches Oligonukleotid der Sequenz 5'-GGCTCACCGCCCACGCTGGA-CATTG-3', entsprechend einer Sequenz aus dem 5'-Ende des bioB-Gens (Otsuka, A. J., Dissertation, University of California, San Diego, CA.; 1978) als Sonde mit der filtergebundenen DNA hybridisiert. Hierzu wurden zunächst 40 pMol dieses Oligonukleotids mit T4-Polynukleotid-Kinase und γ-[³²P]-ATP (75µCi) endmarkiert. Die Hybridisierung der filtergebundenen DNA mit der radioaktiv markierten Sonde erfolgte nach Sambrook et al., (1989, ibid., 9.52-9.55). Hierzu wurde die DNA zunächst 2 h in 5x Denhardt-Lösung (1x Denhardt-Lösung: 0,02% Rinderserumalbumin, 0,02% Ficoll, 0,01% Polyvinylpyrrolidon), 6x SSC-Puffer (1x SSC: 150 mM NaCl, 15 mM Natriumcitrat, pH 7,2) und 150 µg/ml Lachssperm-DNA prähybridisiert, anschließend 18 h in 2x Denhardt-Lösung, 6 x SSC, 0,5% SDS, 150 µg/ml Lachssperm-DNA hybridisiert und 2 h gewaschen und schließlich viermal je 30 min lang in 2x SSC, 0,1% SDS gewaschen. Die Temperatur betrug bei allen Schritten 65°C. Das markierte Oligonukleotid hybridisierte auf diesem "Southern blot" mit einem 5,4 kb langen PstI-Fragment.

Zur Klonierung dieses 5,4 kb-PstI-Fragments mit dem Biotin-Operon wurden zunächst 50 µg der Gesamt-DNA von E. coli DSM 498 mit PstI geschnitten und auf einem 0,7% Agarosegel wie oben aufgetrennt. Fragmente mit einer Größe von 4,5 kb bis 6,5 kb wurden aus dem Gel ausgeschnitten und durch Elektrodialyse in Dialyseschläuchen isoliert. Ungefähr 0,6 µg dieser Fragmente wurden mit 0,6 µg des mit PstI geschnittenen Vektors pHE3 (Hennecke et al., Gene 19:231-234; 1982) ligiert. Dieser Vektor enthält das Gen für die Chloramphenicol-Resistenz (Cm^{R}), das ColE1 Replikon aus pACYC184 (Chang und Cohen, J. Bacteriol., 134:1141-1156; 1978) sowie das E. coli Gen pheS für Phenylalanin-tRNA-Synthetase, das eine PstI-Stelle aufweist.

0,2 ml kompetente Zellen von E. coli RR28 (Hennecke et al., 1982, ibid.) in 50 mM CaCl₂ wurden mit diesem Ligationsansatz transformiert. E. coli RR28 hat im Chromosom ein mutiertes pheS-Gen (pheS12) und ist deshalb resistent gegen p-Fluorphenylalanin (pFphe) im Wachstumsmedium. Wenn RR28 das Plasmid pHE3 mit dem pheS Wildtyp-Gen trägt, ist der Stamm dagegen sensitiv gegen pFphe. Die Insertion von DNA-Fragmenten in die PstI-Schnittstelle von pHE3 unterbricht das pheS Wildtyp-Gen; RR28 mit einem rekombinierten Plasmid ist daher pFphe-resistent (pFphe^{R}). Transformierte Zellen wurden auf pFphe-Minimalmedium (7,1 g/l Na₂HPO₄, 13,6 g/l KH₂PO₄, 0,014 g/l CaCl₂X2H₂O_{'} 0,25 g/l MgSO_{4'} 1,58 g/l (NH₄)₂SO_{4'} 15 g/l Agar, 4 g/l Glucose, 0,005 g/l Thiamin, 0,05 g/l Leucin, 0,05 g/l Prolin, 0,2 g/l D,L-p-Fluorphenylalanin, 0,02 g/l Chloramphenicol; Hennecke et al., 1982, ibid.) plattiert und ca. 2500 Cm^{R} pFphe^{R} Klone wurden isoliert, die das Plasmid pHE3 (Cm^{R}) mit einem Insert im pheS-Gen (pFphe^{R}) enthielten. 600 dieser Klone wurden auf Nitrocellulose-Filter überstempelt, die auf Nutrient Agar (NA)-Platten (NA: Blood Agar Base (Oxoid), 40 g/l; Hefeextrakt (Oxoid), 5 g/l) mit 20 µg/ml Cm lagen. Filter mit angewachsenen Kolonien (3-5 mm Durchmesser) wurden nach Grunstein und Hogness (Proc. Natl. Acad. Sci. USA 72:3961-3965; 1975) behandelt, um die Zellen zu lysieren und die freigesetzte DNA zu binden. Filter mit den lysierten und fixierten E. coli-Zellen wurden mit dem oben beschriebenen 25 Nukleotide langen und mit ³²P markierten bioB-Oligonukleotid hybridisiert. Die Hybridiserung erfolgte gemäß den Modifikationen für die Koloniehybridisierung nach Sambrook et al. (1989, ibid., 11.00), d.h. die Prähybridisierung, die Hybridisierung und der erste Waschvorgang erfolgten in 4x Denhardt-Lösung, 6x SSC, 100 µg/ml Lachssperm-DNA, gefolgt von 6x Waschen in 2x SSC. Die Temperatur betrug 65°C. 3 Klone banden das bioB-Oligonukleotid; aus einem dieser Klone wurde das Plasmid pBO1 mit dem 5,4 kb langen PstI-Fragment (Fig. 2) isoliert. Restriktionsanalysen und ein Vergleich mit publizierten Daten (Szybalski und Szybalski, Gene 19:93-103; 1982) zeigten, daß pBO1 alle Gene des Biotin-Operons enthielt mit Ausnahme von bioD.

Zur Klonierung des bioD-Gens wurde eine Sonde mit Teilen der Gene bioC und bioD verwendet, bestehend aus einem 520 bp langen SphI/PstI-Fragment aus pBO1. Dieses Fragment wurde aus einem Agarosegel isoliert und 0,2 µg des isolierten Fragments wurden mittels "Nick-Translation" mit DNA-Polymerase I (Boehringer Mannheim, BRD; Holoenzym aus E. coli; diese sog. "Kornberg-Polymerase" wurde zusammen mit DNase I verwendet) und 25 µCi α-[³²P]-dATP (NEN-Du Pont, NEG-012H) radioaktiv markiert (Sambrook et al., 1989, ibid. 10.8). Die Hybridisierung dieser Sonde mit durch SspI erzeugten Restriktionsfragmenten des E. coli DSM 498-Chromosoms auf einem "Southern blot" wie oben beschrieben zeigte einerseits das aus pBO1 bekannte 1,6 kb-SspI-Fragment mit bioF und bioC und andererseits ein 1,1 kb-SspI-Fragment mit bioD und Sequenzen des benachbarten Gens uvrB (Sancar et al., Cell, 28; 523-520; 1982).

Zur Klonierung des 1,1 kb-SspI-Fragments wurde wiederum eine partielle Genbank angelegt. Hierzu wurden 30 µg DNA von E. coli DSM 498 mit SspI geschnitten und auf einem 0,7% Agarosegel aufgetrennt. Fragmente der Größe von 0,9 kb bis 1,3 kb wurden ausgeschnitten und durch Elektrodialyse isoliert. 0,5 µg dieser Fragmente wurden mit 0,5 µg des mit SmaI geschnittenen Phagenvektors M13mp19 (Yanisch-Perron et al., 1985, ibid.) ligiert. Mit diesem Ligationsansatz erfolgte eine Transfektion von E. coli JM109 (Yanisch-Perron et al., 1985, ibid) nach Messing (Methods Enzymol., 101:20-79; 1983). 150 Phagenklone mit Insert (Phänotyp LacZ⁻) wurden isoliert und in NYB-Medium vermehrt. Nach Abzentrifugieren der E. coli-Zellen wurden die Phagen in je 50 µl der Überstände mit einer Schleicher & Schüll "minifold I" Apparatur als "dot blot" auf einen Nitrocellulose-Filter (Schleicher & Schüll BA 85) aufgetragen. Zur Denaturierung der Phagen wurden die Filter 5 min mit 0,1 M NaOH/1,5 M NaCl-Puffer behandelt und anschließend mit 0,5 M Tris-HCl, pH 7,5/2,5 M NaCl (5 min) neutralisiert. Die DNA wurde durch Inkubation (2 h) bei 80°C auf dem Filter fixiert. Der Filter wurde wie beschrieben (Sambrook et al., 1989, ibid., 9.52-9.55) mit dem radioaktiv markierten 520 bp langen SphI/PstI-Fragment bei 60°C hybridisiert. Auf diese Weise wurde der Phagenklon M13bioD mit dem oben beschriebenen 1,1 kb-SspI-Fragment, das das bioD-Gen enthält, identifiziert (Fig. 2).

### 1.2 Konstruktion von pBO2

Jeweils 0,5 µg des Plasmids pBO1 und 0,5 µg des Phagen M13bioD wurden mit den Restriktionsenzymen SnoI und HindIII geschnitten und in einem Ansatz religiert. Nach Transformation von E. coli RR28 mit diesem Ansatz wurden rekombinierte Plasmide durch Restriktionsanalyse untersucht. Ein Plasmid, pBO2 (Fig. 2), wurde ausgewählt, in dem ein ca. 1,5 kb langes SnoI/HindIII-Fragment von pBO1, das einen Teil des bioD-Gens und nicht-essentielle Sequenzen des Vektors pHE3 enthält, durch ein 0,95 kb langes SnoI/HindIII-Fragment aus M13bioD ersetzt ist. Eine Analyse zeigte, daß das Plasmid pBO2 das komplette bio-Operon, wie es in E. coli vorliegt, zusammen mit Sequenzen des uvrB-Promotors (Sancar et al., Cell 28:523-530; 1982) stromabwärts von bioD enthielt.

### 1.3 Konstruktion von pBO3 und pBO6

Es wurde beobachtet, daß E. coli RR28 mit pBO2 auf NA-Platten schlechter wächst als mit pBO1 und deutlich kleinere Kolonien bildet. Die Ursache hierfür konnten die uvrB Sequenzen in pBO2 sein. Zur Deletion dieser uvrB Sequenzen wurden 20 µg pBO2-DNA mit HindIII geschnitten und in 150 µl Bal31-Puffer (600 mM NaCl, 12,5 mM MgCl₂, 12,5 mM CaCl₂, 1 mM EDTA, 20 mM Tris-HCl, pH 7,2) aufgenommen. Dann wurde zur schrittweisen Verkürzung der linearen Plasmide Bal31 (aus Alteromonas espejiani, Boehringer Mannheim, BRD) zugegeben. Nach 3, 6, 9, 12 und 15 min Inkubation bei 30°C wurden Aliquots von je 30 µl entnommen und die Bal31-Reaktion durch Zugabe von je 2 µl 0,5 M EGTA (Ethylenglykol-bis-(2-aminoethyl)-tetraessigsäure), pH 7,5, und anschließende Phenolextraktion gestoppt. Dann wurden die Aliquots in 40 µl Mung Bean Nuclease-Puffer (30 mM Natriumacetat, 50 mM NaCl, 1 mM ZnCl₂, 5% Glycerin, pH 4,6) aufgenommen und zur Entfernung ungepaarter Einzelstrangenden und Erzeugung unspezifischer stumpfer Enden 10 min bei 37°C mit Mung Bean Nuclease (Boehringer Mannheim, BRD) behandelt.

Bei der Behandlung mit Bal31 werden nicht nur die uvrB-Sequen-zen, sondern auch essentielle Sequenzen des Vektors pHE3 entfernt. Daher wurden die verkürzten pBO2-Plasmide nach der Behandlung mit Mung Bean Nuclease mit EcoRI geschnitten, um den Teil der Vektor-DNA von pHE3 zu entfernen, der durch Bal31 verkürzt war. Die ursprüngliche Vektorsequenz wurde dann regeneriert, indem die behandelten pBO2-Plasmid mit einem 1,5 kb-DNA-Fragment ligiert wurden, das nach Restriktion mit BamHI, Behandlung mit Mung Bean Nuclease und einer weiteren Restriktion mit EcoRI aus pBO2 isoliert wurde und das die zuvor deletierten essentiellen Vektorsequenzen von pHE3 besitzt. Da durch diese Ligation die Cm-Resistenz des Vektors vollständig regeneriert wird, können intakte Plasmide über ihre Eigenschaft, Resistenz gegen Cm zu vermitteln, erkannt werden.

E. coli RR28 wurde mit den Ligationsansätzen transformiert und auf NA-Platten mit 20 µg/ml Cm plattiert. Man beobachtete kleine, langsam wachsende Kolonien, wie sie für pBO2 typisch sind, und große, normal wachsende Kolonien. Die Anzahl der großen Kolonien pro pBO2-Aliquot nahm mit der Dauer der Bal31-Inkubation zu.

Aus 22 normal wachsenden Kolonien wurde Plasmid-DNA isoliert und durch Restriktions- und Sequenzanalyse untersucht. Auf diese Weise wurden die Plasmide pBO3 und pBO6 erhalten, in denen ca 330 bp bzw. 410 bp der uvrB Region deletiert waren, die aber das bioD-Gen noch vollständig besaßen.

### 1.4 Klonierung der bio-Gene in einer Transkriptionseinheit

### 1.4.1. Konstruktion von pB022: tac Promotor vor bioB

Zum Einbau eines geeigneten Promotors vor das Gen bioB muß der unerwünschte Wildtyp-Promotor vor den Genen bioBFCD entfernt werden. Dies kann durch Schneiden mit NcoI erfolgen, wodurch gleichzeitig das Startkodon des bioB-Gens freigelegt wird. Im vorliegenden Fall wurde als Promotor der tac-Promotor (Russell und Bennett, 1982, ibid.) gewählt, da er als konstitutiver oder induzierbarer Promotor eingesetzt werden kann und nicht nur in E. coli, sondern auch in vielen anderen Gramnegativen Bakterien sehr gute Aktivität hat.

Ein DNA-Fragment mit dem tac-Promotor mit HindIII- und BamHI-Enden wurde von Pharmacia-LKB (Uppsala, Schweden) bezogen und in das mit HindIII und BamHI-geschnittene Plasmid pUC18 (Yanisch-Perron et al., 1985, ibid) eingebaut. Es resultierte das Plasmid pUC18/tac (Fig. 2). 8 µg dieses Plasmids wurden dann mit BamHI geschnitten und zum Auffüllen der rezessiven 3'-Enden mit Klenow-Polymerase (DNA-Polymerase I aus E. coli; Boehringer Mannheim BRD) in Klenow-Polymerase-Puffer (20 mM Tris-HCl, pH 7,5, 10 mM MgCl₂, 6 mM ß-Mercaptoethanol) unter Zusatz von je 100 µM dATP, dGTP, dCTP und dTTP inkubiert. Anschließend erfolgte eine zweite Restriktion mit AatII. Auf diese Weise konnte ein 0,55 kb langes DNA-Fragment mit dem tac-Promotor isoliert werden.

Ein 3,2 kb-Fragment mit den Genen bioB, bioF, bioC und dem 5'-Ende des bioD-Gens wurde aus pBO1 isoliert. Dazu wurden 8 µg pBO1 mit NcoI geschnitten und anschließend zum Auffüllen der rezessiven 3'-Enden wie oben mit Klenow-Polymerase behandelt. Anschließend erfolgte eine zweite Restriktion mit PstI, gefolgt von der Isolierung des gewünschten 3,2 kb-Fragments. Schließlich wurden 4 µg des Vektors pHE3 mit PstI und AatII geschnitten und das P15A-Replikon aus pHE3 (Hennecke et al., 1982, ibid.) isoliert.

Diese drei Fragmente wurden zur Ligation der überstehenden und glatten Enden in einem Ansatz in äquimolaren Mengen mit T4-DNA-Ligase (Boehringer Mannheim, BRD) behandelt, wobei jeweils die überstehenden Enden von PstI mit PstI und von AatII mit AatII und die nach der Behandlung mit Klenow-Polymerase glatten Endenvon BamHI und NcoI miteinander ligiert wurden. Bei der Ligation des mittels Klenow-Polymerase aufgefüllten BamHI-Endes mit dem ebenso behandelten NcoI-Ende werden die BamHI-und NcoI-Schnittstellen regeneriert. E. coli RR28 wurde mit diesem Ligationsansatz transformiert und auf Cm^{R} selektioniert. Die Plasmid-DNA von Transformanten mit Cm^{R} wurde durch Restriktionsanalyse untersucht. Auf diese Weise wurde das Plasmid pBO21 (Fig. 2) erhalten, in dem sich der tac-Promotor vor dem bioB-Gen befindet. Durch Deletion eines 1,5 kb langen HindIII-Fragments aus pBO21, das nicht-essentielle Sequenzen aus den Plasmidvektoren pHE3 und pUC18 aufweist, wurde schließlich pBO22 erhalten (Fig. 2).

### 1.4.2. Konstruktion von pBO27 und pB028

5 µg pB022 wurden mit PstI geschnitten und das überhängende PstI-Ende durch Behandlung mit Mung Bean Nuclease zu einem glatten Ende verkürzt. Dann wurde mit SnoI geschnitten und das resultierende 6,8 kb lange DNA-Fragment isoliert. Ein 0,76 kb-DNA-Fragment mit dem 3'-Ende des bioD-Gens wurde aus 5 µg pBO3 nach Restriktion mit ClaI, Auffüllen der überhängenden ClaI-Enden mittels Klenow-Polymerase und Restriktion mit SnoI isoliert. Die beiden DNA-Fragmente wurden mittels T4-DNA-Ligase ligiert und dann wurde E. coli mit dem Ligationsansatz transformiert. Nach Selektion auf Chloramphenicol wurde aus den Transformanten mit Cm^{R} nach Restriktionsanalyse das Plasmid pBO27 erhalten. Dieses Plasmid enthält den tac-Promo tor zusammen mit den Genen bioB, bioF, bioC und dem vollständigen bioD-Gen in einer Transkriptionseinheit (Fig. 2).

Zur Deletion der BamHI Schnittstelle in pBO27 wurden 5 µg pBO27 mit BamHI geschnitten, mit Klenow-Polymerase und dem Nukleotid dGTP wie oben beschrieben inkubiert und dann mit Mung Bean Nuclease behandelt. Nach Religation dieser DNA mit T4-DNA-Ligase und Transformation von E. coli DH5 (Hanahan, J. Mol. Biol. 166:557-580; 1983) wurde Plasmid pBO28 erhalten, in dem die BamHI Schnittstelle deletiert ist, während die NcoI Schnittstelle erhalten geblieben ist (Fig. 2).

### 1.4.3. Konstruktion von M13bio18 und M13bio18/13

Zur Entfernung des unerwünschten Wildtyp-Promotors vor dem bioA-Gen wurde zunächst durch Restriktion von 5 µg pBO3 mit bglII und KPnI ein 4,4 kb-Fragment mit den Genen bioB, bioF, bioA und ORFI isoliert. 0,5 µg dieses Fragments wurden mit 0,5 µg des mit BamHI und KpnI geschnittenen Phagenvektors M13mp18 (Yanisch-Perron et al., 1985, ibid.) ligiert. Nach Transformation von E. coli JM109 (Yanisch-Perron et al., 1985, ibid.) mit diesem Ligationsansatz wurden rekombinierte Phagenklone, die ein Insert besaßen, nach Messing (1983, ibid.) identifiziert; doppelsträngige Phagen-DNA aus solchen Klonen wurde isoliert und mittels Restriktionsanalyse untersucht. Auf diese Weise wurde der Phage M13bio18 mit dem gewünschten 4,4 kb-Fragment erhalten (Fig. 2).

25 µg doppelsträngiger DNA des Phagen M13bio18 wurden durch Restriktion mit NcoI linearisiert, in 160µl Bal31-Puffer aufgenommen, und anschließend wurde Bal31 zur Entfernung des bioA-Promotors zugegeben. Nach 20, 40, 60, 80, 100 und 120 Sekunden Inkubation bei Raumtemperatur wurden Aliquots von je 25µl entnommen und die Bal31-Reaktion durch Zugabe von 2µl 0,5 M EGTA, pH 7,5, und Phenolextraktion gestoppt. Je 3 Aliquots wurden vereinigt und mit XbaI geschnitten, um die Gene bioB und bioF zu entfernen. Anschließend wurde die DNA mit Klenow-Polymerase wie oben behandelt, um überstehende 5'-Enden zu glatten Enden aufzufüllen. Die so behandelte DNA wurde religiert und E. coli JM109 wurde mit der ligierten DNA transformiert. Von 24 Phagenklonen wurde einzelsträngige DNA isoliert (Messing, 1983, ibid.) und die DNA-Sequenz am 5'-Ende des bioA Gens wurde nach Sanger et al. (1977; ibid.) analysiert. Auf diese Weise wurde der Phagenklon M13bio18/13 erhalten, in dem der Wildtyp-Promotor vor dem bioA-Gen deletiert ist und der gleichzeitig eine SalI-Schnittstelle 26bp stromaufwärts des bioA-Gens aufweist (Fig. 2).

### 1.4.4. Konstruktion von M13bioDA

Zur Anordnung der Gene bioD und bioA in einer Transkriptionseinheit wurden 5µg des Plasmids pBO6 (Fig. 2) mit SphI und SalI geschnitten. Das resultierende 0,97 kb lange DNA-Fragment, das das Gen bioD und 72bp der DNA stromabwärts des bioD-Gens bis zum SalI-Ende enthält, wurde isoliert. Ebenfalls mit SphI und SalI geschnitten wurden 2 µg von M13bio18/13. Die DNA-Fragmente wurden mit T4-DNA-Ligase ligiert und E. coli JM109 wurde transformiert. Aus 24 rekombinierten Klonen wurde doppelsträngige Phagen-DNA isoliert und mittels Restriktionsanalyse charakterisiert. Auf diese Weise wurde der Klon M13bioDA erhalten, in dem die Gene bioD und bioA 98bp voneinander entfernt sind (Fig. 2).

### 1.4.5. Konstruktion von pBO30

Zur Konstruktion einer Transkriptionseinheit mit dem tac-Promotor vor den bio-Genen wurden 5µg DNA von M13bioDA mit EcoRI geschnitten, zum Auffüllen überhängender EcoRI-Enden wie oben mit Klenow-Polymerase behandelt und dann mit SnoI geschnitten. Das resultierende 2,6 kb lange DNA-Fragment mit den Genen bioD, bioA und ORFI wurde isoliert. 5 µg des Plasmids pB028 (Fig. 2) wurden mit SalI geschnitten, zur Abspaltung überhängender SalI-Enden mit Mung Bean Nuclease behandelt und dann ebenfalls mit SnoI geschnitten. Ein 6,7 kb langes DNA-Fragment mit Vektor-DNA, tac-Promotor und den Genen bioBFC wurde isoliert.

Die isolierten DNA-Fragmente wurden mit T4-DNA-Ligase ligiert und der Biotin-auxotrophe Stamm E. coli SA291 (Cleary und Campbell, J. Bacteriol. 112:830-839; 1972) wurde mit diesem Ligationsansatz transformiert. Durch Ausplattieren auf NA-Platten mit 20µg/ml Cm und 8µg/ml Avidin wurden Klone mit einem kompletten Biotin-Operon im Plasmid selektioniert. Plasmide aus solchen Klonen wurden mittels Restriktionsanalyse überprüft. Auf diese Weise wurde Plasmid pB030 erhalten, das die Gene bioB, bioF, bioC, bioD und bioA und das Gen ORFI zusammen mit dem tac-Promotor in einer Transkriptionseinheit enthält (Fig. 2).

### 1.5 Konstruktion von Plasmiden mit verbesserter Expression der bio-Gene

### 1.5.1. Konstruktion von pBO30A-9 und pB030A-15

In Minizellen von E. coli DS410 (Dougan und Sheratt, Mol. Gen. Genet. 151: 151-160; 1977) mit dem Plasmid pBO30 war die von dem bioA-Gen kodierte DAPA-Aminotransferase wesentlich schwächer exprimiert als die anderen Enzyme für die Biotin-Synthese. In einem Versuch, die Expression des bioA-Gens zu verbessern, wurde der Abstand zwischen dem bioD-Gen und dem bioA-Gen mit Exonuklease Bal31 verkürzt, um mögliche störende Sequenzen wie "stem-loop"-Strukturen zu entfernen. Dazu wurden 25µg pBO30 mit SalI geschnitten und dann wie oben beschrieben mit Exonuklease Bal31 und mit Klenow-Polymerase behandelt. Durch Ligation mit einem synthetischen Oligonukleotid der Sequenz 5'-CGTCGACG-3', einem SalI-Linker, wurde die SalI-Schnittstelle regeneriert. Dann wurde die DNA mit SalI und SnoI geschnitten und die am 3'-Ende verkürzten bioD-Fragmente mit einer Länge von etwa 640 bp isoliert. Diese Fragmente wurden mit einem 8,25 kb großen Fragment aus pBO30 ligiert, das nach Schneiden dieses Plasmids mit SalI und SnoI isoliert werden konnte und das unveränderte bioA-Gen enthält.

Der Biotin-auxotrophe Stamm E. coli SA291 wurde mit obigem Ligationsansatz transformiert, um dann auf NA-Platten mit 60µg/ml Cm und 5µg/ml Avidin Klone mit einem intakten bioD-Gen zu selektionieren. Man erhielt 26 solcher Klone, die durch Restriktionsanalyse untersucht wurden. 8 dieser Klone mit offensichtlicher Verkürzung der Region stromaufwärts von der SalI-Stelle wurden durch DNA-Sequenzanalyse genauer charakterisiert. In fünf dieser Klone waren wie gewünscht etwa 20 bis 45 bp der DNA zwischen dem bioD- und dem bioA-Gen deletiert. In E. coli-Minizellen bewirkten diese Klone tatsächlich eine gegenüber pB030 um den Faktor 2 erhöhte Expression des bioA-Gens. Ein Beispiel für ein Plasmid mit derart verbesserter Expression ist das auf diese Weise erhaltene Plasmid pBO30A-9 (Fig. 3).

Überraschend wurden drei weitere Plasmide isoliert, in denen 70 bis 90 bp der DNA zwischen dem bioD-Gen und dem bioA-Gen deletiert waren. Die Deletionen reichten also bis in das bioD-Strukturgen. Daraus resultierte (i) ein jeweils unterschiedlicher COOH-Terminus der DTB-Synthetase ohne große Veränderung der Enzymaktivität und (ii) eine Überlappung der veränderten bioD-Gene mit dem bioA-Leseraster. Auf diese Weise wurde beispielsweise Plasmid pB030A-15 mit der bioD-Gen-Mutante bioDl5 erhalten (Fig. 3, 5 und 6). In E. coli-Minizellen mit pBO3-0A-15 ist die bioA-Expression im Vergleich zu pB030 um den Faktor 4 erhöht.

Die DNA-Sequenzen der bioDA-Region und die daraus abgeleiteten Aminosäuresequenzen der Plasmide pBO30, pBO30A-9 und pBO30A-15 sind in Fig. 3 dargestellt (Seq ID No 9-16).

### 1.5.2 Konstruktion von Plasmiden mit verbesserter Ribosomenbindungsstelle vor dem bioB-Gen

Zur Verbesserung der Translation des bioB-Gens, dessen Expression in pB030 deutlich schwächer ist als beispielsweise die des bioD-Gens, wurde die Sequenz stromaufwärts des bioB-Gens in pB030, die den tac-Promotor und eine Ribosomenbindungsstelle umfaßt, die in dem klonierten tac-Promotor-Fragment enthalten ist, modifiziert. Hierzu wurden synthetische, sogenannte gemischte oder "mixed" Oligonukleotide mit variablen Sequenzen vor das Gen bioB gesetzt. Zur einfachen Auswahl günstiger Ribosomenbindungsstellen wurde ein Testplasmid mit einer translationellen bioB::lacZ Genfusion, pbioB::lacZ-2, verwendet. pbioB::lacZ-2 ist im Vektorteil, im tac-Promotor mit der Ribosomenbindungsstelle und im 5'-Ende des Gens bioB identisch mit dem Plasmid pB022 (Fig. 2). An einer NruI-Schnittstelle nach Nukleotid 326 des bioB-Strukturgens wurden aber das 3'-Ende des bioB-Gens und die restlichen bio-Gene deletiert und das lacZ-Gen von E. coli (Casadaban et al., Methods Enzymol. 100:293-308; 1983) so eingebaut, daß bioB und lacZ im korrekten Leseraster zur Expression eines bioB::lacZ Fusionsproteins fusioniert waren, und daß die NruI-Schnittstelle regeneriert wurde.

In das Plasmid pbioB::lacZ-2 wurde in mehreren Schritten das Oligonukleotid 985E mit der Sequenz 5'-CATGGAATCCTCCACTGATCAG-TAC-3' vor das bioB-Gen eingefügt (Fig 4). Hierzu wurde pbioB::lacZ-2 zunächst mit BamHI gespalten und dann die überhängenden BamHI-Enden wie beschrieben mit Klenow-Polymerase aufgefüllt. Bei diesen Schritten wurde offensichtlich ein Guanin-Rest (G) unspezifisch deletiert, was den Verlust einer BamHI-Schnittstelle in den späteren Plasmiden zur Folge hatte. Nach Einfügen eines KpnI-Linkers wurde E. coli XL1-Blue (Bullock et al., Biotechniques 5:376-379; 1987) mit dem Ligationsansatz transformiert und das Plasmid pbioB::lacZ/KpnI isoliert. Dieses Plasmid wurde mit NcoI partiell geschnitten und dann mit KpnI nachgeschnitten. Nach Ligation mit dem Oligonukleotid 985E wurde der zweite DNA-Strang mit Klenow-Polymerase aufgefüllt. Nach Transformation von E. coli XL1-Blue und Selektion auf NA-Platten mit 20µg/ml Cm, 30µq/ml X-Gal und 0,5 mM IPTG (Isopropylthiogalactosid) konnte das Plasmid pbioB::lacZ/985E isoliert werden (Fig. 4). Plasmid pbioB::lacZ/985E wurde weiter variiert, indem die Ribosomenbindungsstelle durch Restriktion mit KpnI und SpeI ausgeschnitten und durch drei verschiedene gemischte Oligonukleotide, SD17, SD19 und SD21, ersetzt wurde (Fig. 4). Nach Ligation mit diesen Oligonukleotiden wurde durch Inkubation mit Klenow-Polymerase die Lücke im zweiten DNA-Strang geschlossen. Bakterienzellen von E. coli XL1-Blue wurden mit dieser DNA transformiert und wie oben auf NA-Platten mit 20µg/ml Cm, 30µg/ml X-Gal und 0,5 mM IPTG plattiert. 20 Klone mit guter Expression des bioB::lacZ-Fusionsproteins, die auf diesem Medium dunkelblaue Kolonien bildeten, wurden ausgewählt und die β-Galaktosidase-Aktivität dieser Klone wurde mit einem Enzym-Assay nach Miller (Experiments in Molecular Genetics, Cold Spring Harbor Laboratory, Cold Spring Harbor, N.Y., S. 352-355; 1972) gemessen. Hierzu wurden zuvor die E. coli-Stämme mit bio::lacZ-Plasmiden in Flüssigkultur bis zu einer Optischen Dichte bei 600 nm (OD₆₀₀) von ca. 0,5 angezogen.

Die höchste β-Galaktosidase-Aktivität zeigten die Plasmide pbioB::lacZ/985E, pbioB::lacZ/16 und pbioB::lacZ/9 (Fig. 4), bei denen die β-Galaktosidase-Aktivität im Vergleich zu pbioB::lacZ-2 um den Faktor 2,1, 3,4 bzw. 5,9 gesteigert war. Die DNA-Sequenz der optimierten Ribosomenbindungsstellen für das bioB-Gen in diesen Plasmiden wurde nach Sanger et al. (1977, ibid.) bestimmt.

Zum Einbau der optimierten Ribosomenbindungsstellen in eine Transkriptionseinheit mit den bio-Genen wurden jeweils 5µg der Plasmide pbioB::lacZ/985E, pbioB::lacZ/16 oder pbioB::lacZ/9 mit ClaI und NruI geschnitten und ein ca. 550bp langes DNA-Fragment mit dem tac-Promotor, der jeweiligen Ribosomenbindungsstelle und dem 5'-Ende des bioB-Gens isoliert. Gleichzeitig wurden 5µg des Plasmids pBO30ΔA (Fig. 5) mit ClaI und NruI geschnitten und ein 7,7 kb langes DNA-Fragment isoliert. In pB030Δ A, das sich von pB030 ableitet, ist ein SalI/BamHI-Fragment mit dem Großteil des bioA-Gens und einer störenden NruI-Schnittstelle deletiert (Fig. 5). Die beiden Fragmente wurden ligiert und Klone mit rekombinierten Plasmiden wurden isoliert. Auf diese Weise wurden die Plasmide pB030 ΔA/9, pBO30ΔA/16 und pBO30ΔA/985 erhalten. Fig. 5 zeigt eine solche Konstruktion am Beispiel von pBO30ΔA/9 mit der Ribosomenbindungsstelle aus pbioB::lacZ/9.

Je 2µg der Plasmide pBO30ΔA/9, pBO30ΔA/l6 und pBO30ΔA/985E wurden mit SnoI und KpnI geschnitten, wobei KpnI in geringer Menge eingesetzt wurde, um nur partiell zu schneiden. Dann wurden jeweils 6,6 kb lange DNA-Fragmente isoliert, die die Vektor-DNA, den tac-Promotor, das bioB-Gen mit der verbesserten Ribosomenbindungsstelle und die Gene bioFC enthielten. Das Plasmid pB030A-15 (4 µg) wurde ebenfalls mit SnoI und NcoI geschnitten und ein 2,8 kb-Fragment mit den Genen bioDA-ORFI isoliert. Die isolierten Fragmente wurden ligiert und E. coli RR28 wurde mit der Ligationsmischung transformiert. Rekombinierte Plasmide mit einem kompletten Biotin-Operon wurden durch Restriktionsanalyse identifiziert. Auf diese Weise wurden die Plasmide pBO30A-15/9, pB030A-15/16 und pBO30A-l5/985E erhalten. Diese enthalten alle die optimierte bioDA-Region aus pB030A-15 mit den entsprechenden optimierten Ribosomenbindungsstellen aus den Plasmiden pbioB::lacZ/9, pbioB::lacZ/16 bzw. pbioB::lacZ/985E. Die genetischen Kontrollelemente dieser Plasmide, nämlich die Kombination aus tac-Promotor und optimierter Ribosomenbindungsstelle, die in direkter Verknüpfung mit dem bioB-Gen stehen und dessen effiziente Expression bewirken, weisen die folgenden Sequenzen auf: Fig. 5 zeigt die Konstruktion von Plasmiden, die die Gene bioB, bioF, bioC, bioD und bioA zusammen mit einer optimierten Ribosomenbindungsstelle enthalten, am Beispiel der Konstruktion von pBO30A-l5/9.

Die gesamte Transkriptionseinheit der bio-Gene in pB030A-15/9 wurde sequenziert. Die Sequenz und die davon abgeleiteten Genprodukte sind in Fig. 6 dargestellt (Seq ID No: 1 - 8)

### 1.6 Konstruktion von pBO30A-15/9ΔorfI

2µg des Plasmids pBO30ΔA/9 wurden wie oben mit SnoI und KpnI geschnitten und das 6,6 kb lange DNA-Fragment wurde isoliert. 4µg des Plasmids pB030A-15 wurden mit SspI geschnitten. Durch Ligation der resultierenden linearen DNA mit einem KpnI-Linker der Sequenz 5'-CGGTACCG-3' wurde stromabwärts des bioA-Gens eine neue KPpI-Stelle eingefügt. Nach Schneiden mit SnoI wurde ein 2,1 kb-Fragment mit den Genen bioDA isoliert. Die isolierten DNA-Fragmente wurden ligiert und E. coli RR28 wurde mit der Ligationsmischung transformiert. Rekombinierte Plasmide mit den Genen biOBFCDA wurden durch Restriktionsanalyse identifiziert. Auf diese Weise wurde pBO30A-15/9ΔorfI mit einer Deletion des ORFI-Gens erhalten (Fig. 5).

### 1.7 Konstruktion von Plasmid pBO47

5 µg des Plasmids pB030A-15/9 wurden mit den Restriktionsenzymen XbaI und EcoRI geschnitten. Das resultierende 5,8 kb große Restriktionsfragment mit dem tac-Promotor und dem Biotin-Operon wurde isoliert und anschließend mit dem "broad-host-range" Plasmid pRK290X (Alvarez-Morales et al., Nucl. Acid. Res. 14, 4207-4227, 1986; modifiziert durch Deletion einer XhoI Restriktionsstelle und Einfügen einer XbaI-Stelle an derselben Position) ligiert, das ebenfalls mit XbaI und EcoRI geschnitten war. Mit dem Ligationsansatz wurde E. coli S17-1 (Simon et al., Biotechnology 1:784-791; 1983) transformiert. Rekombinierte Plasmide wurden durch Restriktionsanalyse charakterisiert; auf diese Weise wurde Plasmid pBO47 erhalten, das das Biotin-Operon integriert in pRK290X enthält. Durch Konjugation mit dem Stamm E. coli S17-1/pBO47 wurde das Plasmid pBO47 in die Bakterienstämme Rhizobium/Agrobacterium sp. HK4, Pseudomonas mondocina, Pseudomonas aeruginosa PA01 (Holloway, J. Gen. Microbiol. 13:572-581; 1955) und Acinetobacter calcoaceticus DSM 588 transferiert.

### 1.8 Konstruktion von pBO74ΔB

Die Konstruktion von Plasmid pBO74ΔB mit einer Deletion des bioB-Gens erfolgte ausgehend von Plasmid pBO74-13 (Fig. 7). Plasmid pBO74-13 besteht aus den gleichen DNA-Bausteinen wie pBO30 (Fig. 2). Die Reihenfolge der bio-Gene innerhalb des Plasmids pBO74-13 ist jedoch verschieden.

5µg des Plasmids pBO74-13 wurden mit SmaI geschnitten. Nach Extraktion mit Phenol/Chloroform wurde die Plasmid-DNA mit SphI geschnitten und ein 6 kb-Fragment enthaltend die Vektor-DNA, den tac-Promotor, die Gene bioA-ORFI und bioCD wurde isoliert. 18µg des Plasmids pBO3 (Fig. 2) wurden mit SspI und SphI geschnitten und ein 1,66 kb-Fragment mit dem bioF-Gen und einem Teil des bioC-Gens isoliert. Die isolierten Fragmente wurden miteinander ligiert und E. coli RR28 wurde mit der Ligationsmischung transformiert. Rekombinierte Plasmide wurden mittels Restriktionsanalyse analysiert. Auf diese Weise wurde Plasmid pBO74ΔB erhalten, das sich von Plasmid pBO74-13 durch die Deletion des bioB-Gens unterscheidet (Fig. 7).

### Beispiel 2

### In vivo Fermentationen von Biotin

### 2.1 In vivo Fermentation von Biotin mit Escherichia coli Produktions-Stämmen

Zellen des E. coli-Stammes XL1-Blue mit pBO30A-15/9 (DSM 7246) wurden mit einem 20 1 MBR-Fermenter in Glycerin-Minimal-Medium (3% Glycerin bei Start der Kultur) in einem fed batch Verfahren über 30 h lang bei 37°C bis zu einer optischen Dichte bei 650 nm (OD₆₅₀) von 20 angezogen. Die Anwesenheit von Plasmid pBO30A-15/9 wurde durch Zugabe von Chloramphenicol (50 µg/ml) in der Vorkultur (3 1 Glycerin-Minimal-Medium) und der batch-Phase der Fermentation sichergestellt. Die Verwendung anderer Kohlenstoffquellen wie Glucose oder Succinat (0,4 % bei Beginn der batch-Fermentations-Phase) war ebenso praktikabel. Die metabolische Aktivität der Zellen wurde anhand ihrer spezifischen Sauerstoff-Aufnahmerate verfolgt. Die Produktion von Biotin während der Fermentation wurde durch Titration der Biotin-Werte des Fermenter-Mediums mit einem Bioassay mit Lactobacillus plantarum verfolgt (E. DeMoll und W. Shive, Anal. Chem. 158:55-58, 1986).

Die Kohlenstoffquelle, in diesem Fall eine 50%-ige Glycerinlösing in deionisiertem Wasser, wurde mit variabler, der jeweiligen Biomasse-Entwicklung angepasster Zuflussrate zugeführt. Für Glycerin wurde ein empirischer Wert von 2 g Glycerin pro Liter Kultur für einen OD-Zuwachs von OD 1 auf OD 2 für die "feed"-Rate zugrunde gelegt.

Der pH des Fermenters wurde durch Zupumpen von40 %-iger H₃PO₄ bzw. 25 %-igem NH₃ automatisch auf pH 7 eingeregelt. Die Belüftung wurde durch Einblasen von 10-25 NL/min Luft und Rotation des Rührers mit 300-700 rpm entsprechend der jeweiligen Biomasse-Entwicklung geregelt. Eine Sauerstoffsättigung zwischen 15 und 40 % wurde angestrebt. Der Sauerstoff- und der CO₂-Gehalt der Abluft wurde paramagnetisch bzw. mittels Infrarot gemessen. Die Temperatur des Fermenters wurde auf 37°C eingeregelt. Bei 37°C wuchs die Kultur mit einer Verdopplungszeit von 2,5 Stunden bis zu einer OD₆₅₀ von 20 und wurde dann stationär.

Während der Fermentation häuften sich innerhalb von 25 Stunden 35 mg/l D(+)-Biotin an. In E. coli-Stämmen ist eine nennenswerte Biotin-Synthese nur in wachsenden Kulturen zu erreichen.

Als weitere geeignete Produktionsstämme erwiesen sich E. coli ED8767 (N. E. Murray et al., Mol. Gen. Genet. 150:53-61; 1975) mit pBO30A-15/9 (DSM 8554) oder E. coli BM4062 (D. F. Barker und A. M. Campbell, J. Bacteriol. 143:789-800; 1980) mit pBO30A-15/9 (DSM 7247).

In ähnlicher Weise wurden die Plasmide pBO3, pBO30 und pBO30A-15/9ΔORFI getestet und die Biotinproduktivität bestimmt. Die nachfolgende Tabelle I zeigt die starke Verbesserung der Biotin-Produktivität von Stämmen mit den Plasmiden pB030, pBO30A-15/9 und pBO30A-15/9ΔORFI, die die bio-Gene in einer Transkriptionseinheit besitzen, im Vergleich mit E. coli S17-1 (Wildtyp, Biotin-Gene auf dem Chromosom) und E. coli S17-1/pBO3 (Biotin-Gene auf dem Plasmid, jedoch divergente Transkription wie im Wildtyp-Operon). Die Versuche zeigen ferner, daß das Fehlen des ORFI-Gens keinen Einfluß auf die Biotin-Produktivität hat.

**Tabelle I**

| Stamm | Biotin-Produktivität pMol/min x 10⁹ Zellen |
|---|---|
| E. coli S17-1 | 0,01 - 0,02 |
| E. coli S17-1/pBO3 | 0,02 - 0,04 |
| E. coli BM 4062/pBO30 | 3,0 - 5,0 |
| E. coli XL1 Blue/pBO30A-15/9 | 10,0 - 20,0 |
| E. coli BM 4062/pBO30A-15/9ΔORFI | 10,0 - 20,0 |

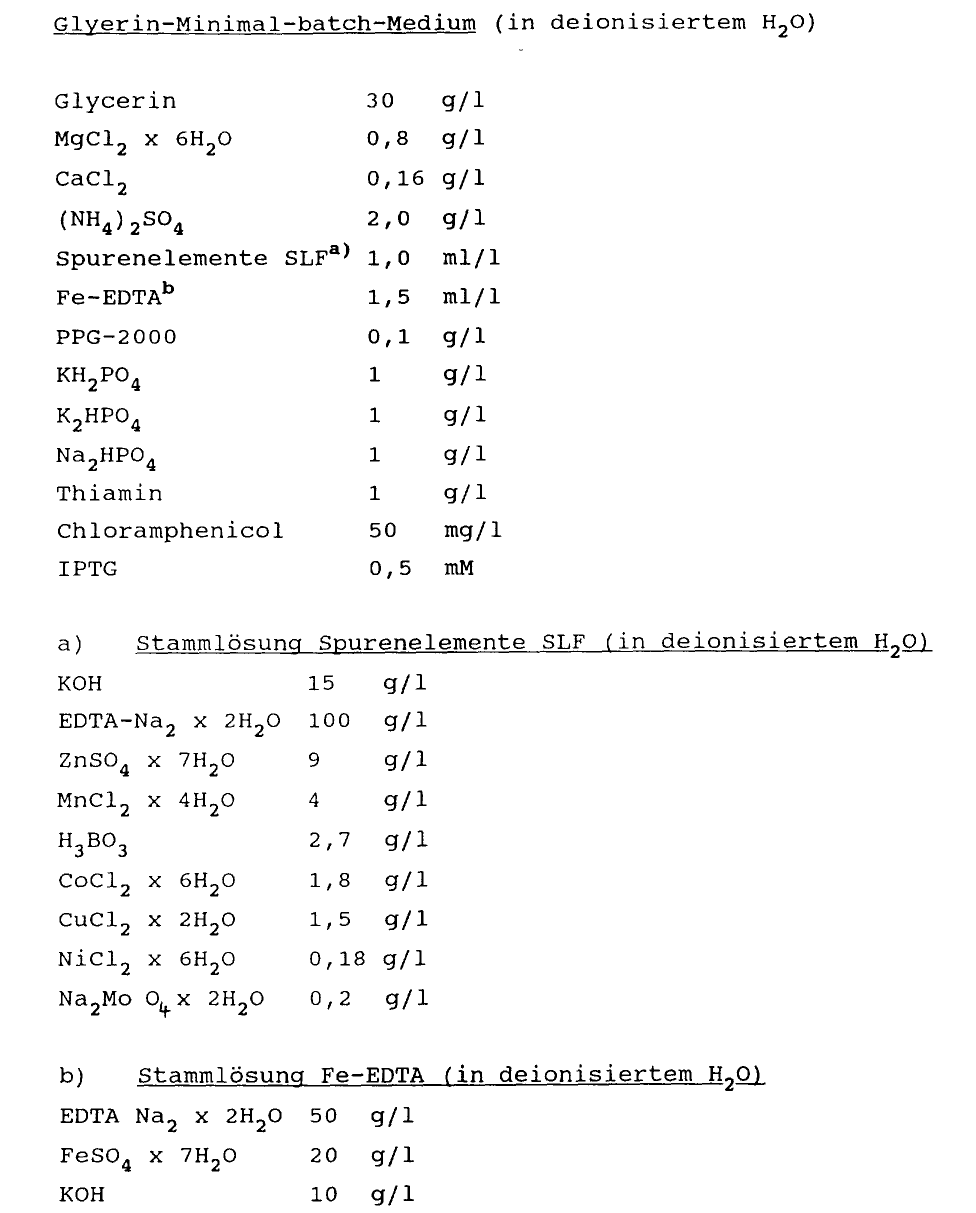

### Antibiotika-Supplemente: (Endkonzentrationen)

100 µg/ml Ampicillin (Natriumsalz, Fluka) und 50 µg/ml Chloramphenicol (Fluka).

### 2.2 In vivo Fermentation von Biotin mit dem Agrobacterium/ Rhizobium-Produktions-Stamm HK4/pBO47

Zellen des Biotin-auxotrophen Stammes Agrobacterium/ Rhizobium sp HK4 mit dem Biotin-Produktions-Plasmid pBO47 (DSM 8555) wurden in einem 2 1 MBR-Fermenter in einem L-Glutamin-säure/Betain Minimalmedium in einem fed-batch Verfahren bei 30°C bis zu einer OD₆₅₀ von 70 angezogen. HK4/pBO47 ist durch eine bemerkenswert stabile Biotin-Synthese-Rate auch bei extrem langsamen Wachstum ("maintenance growth") gekennzeichnet. Deshalb schloss sich in diesem Experiment nach der Anzucht der Biomasse eine lange maintenance Phase (500 Stunden) bei stark reduziertem Kohlenstoff-"feed" an.

Nach der exponentiellen Wachstumsphase, nach Erreichen einer OD₆₅₀ von 12, wurde ein Glucose-Betain-"feed" (360 g/l Glucose plus 103 g/l Betain gelöst in deionisiertem Wasser) langsam dosiert (1,5 ml/Stunde) zugefüttert, um langanhaltendes langsames Wachstum bzw. "maintenance growth" zu ermöglichen. Zum Zeitpunkt 150 Stunden wurde bis zu einer Endkonzentration von 100 mg/l im Fermenter Fe²⁺-Gluconat nachgefüttert. Zu den Zeitpunkten 200, 360 und 550 Stunden wurden 10 ml Salz-Lösung und 1,36 ml Standard-Vitamin-Lösung nachgefüttert.

Der pH des Fermenters wurde durch Zupumpen von 85%-iger Phosphorsäure bzw. 3M Kalilauge automatisch auf pH 7 eingeregelt. Die Belüftung wurde durch Einblasen von 1-3 NL/min Luft und Rotation des Rührers mit 300-1000 rpm entsprechend der jeweiligen Biomasseentwicklung geregelt, so dass eine Sauerstoffspannung von 1-4 mg/l gewährleistet war. Die Temperatur des Fermenters wurde auf 30°C eingeregelt. Die Kultur wuchs in der exponentiellen Wachstumsphase mit einer Verdopplungszeit von 5,6 Stunden, in der Phase mit stark limitiertem "feed" mit einer Verdopplungszeit von 300 Stunden und schwenkte dann zu "maintenance growth" um.

Zu Beginn der Fermentation, nach 200 Stunden und nach 415 Stunden wurde Diaminopelargonsäure (DAPA; zweimal zu einer Endkonzentration von 200 µg/ml, zuletzt zu einer Endkonzentration von 100µg/ml) zur Kultur zugegeben. HK4 selbst ist Biotin auxotroph. Der Stamm war in der Lage aus dem Biotin-Vorläufer DAPA Dethiobiotin zu erzeugen und dieses schließlich mit hoher Ausbeute in D(+)-Biotin umzusetzen. Es wurden 110 mg/l D(+)-Biotin angehäuft. Bemerkenswert hieran ist, dass diese Synthese zum überwiegenden Anteil von nichtwachsenden Zellen vollzogen wurde.

### Glutaminsäure/Betain-Minimalmedium

In 1,25 Liter deionisiertem Wasser wurden gelöst, bzw. dazu zugegeben:

| | |
|---|---|
| 31,25 g | L-Glutaminsäure Mononatriumsalz x H₂O |
| 12,5 g | Betain |
| 0,2 g | CaCl₂ |
| 1,0 g | MgCl₂ x 6 H₂O |
| 1,25 g | K₂SO₄ |
| 1,25 ml | Spurenelemente SLF (Beispiel 2.1) |
| 1,87 ml | Fe-EDTA (Beispiel 2.1) |
| 0,25 ml | Tetracyclin (10 mg/ml in 70% Ethanol) |

| Salz-Lösung | |
|---|---|
| 0,03 g | CaCl₂ |
| 0,16 g | MgCl₂ x 6 H₂O |
| 0,2 g | K₂SO₄ |
| 200 µl | SLF (Beispiel 2.1) |
| 300 µl | HCl konz. |
| (gelöst in 10ml deionisiertem H₂O) | |

| Standard-Vitaminlösung (in deionisiertem H₂O) | |
|---|---|
| 10 mg/l | Pyridoxalhydrochlorid |
| 5 mg/l | Riboflavin |
| 5 mg/l | Nicotinsäureamid |
| 5 mg/l | Thiamin-hydrochlorid |
| 2 mg/l | Biotin |
| 5 mg/l | Pantothensäure |
| 5 mg/l | 4-Aminobenzoesäure |
| 2 mg/l | Folsäure |
| 5 mg/l | Vitamin B12 |

## Patentansprüche

1. DNA-Fragment, das die Gene für die Biotin-Biosynthese bioB, bioF, bioC, bioD und bioA aus Enterobakterien oder deren funktionell äquivalente genetische Varianten und Mutanten umfaßt, dadurch gekennzeichnet, daß die Gene in einer Transkriptionseinheit und in einer Transkriptionsrichtung angeordnet sind und von einem gemeinsamen Promotor transkribiert werden.

2. DNA-Fragment nach Anspruch 1, worin die Enterobakterien ausgewählt sind aus der Gruppe der Gattungen Escherichia, Salmonella und Citrobacter.

3. DNA-Fragment nach Anspruch 1 oder 2, worin die Enterobakterien ausgewählt sind aus der Spezies Escherichia coli.

4. DNA-Fragment nach einem der Ansprüche 1 bis 3, worin der gemeinsame Promotor der tac-Promotor ist.

5. DNA-Fragment nach einem der Ansprüche 1 bis 4, worin das genregulatorische Element der Transkriptionseinheit in direkter Verknüpfung mit dem bioB-Gen die Sequenz umfaßt:

6. DNA-Fragment nach einem der Ansprüche 1 bis 4, worin das genregulatorische Element der Transkriptionseinheit in direkter Verknüpfung mit dem bioB-Gen die Sequenz umfaßt:

7. DNA-Fragment nach einem der Ansprüche 1 bis 4, worin das genregulatorische Element der Transkriptionseinheit in direkter Verknüpfung mit dem bioB-Gen die Sequenz umfaßt:

8. DNA-Fragment nach einem der Ansprüche 1 bis 7, worin der Abstand zwischen den in der Transkriptionseinheit aufeinanderfolgenden Genen bioD und bioA nicht mehr als 50bp beträgt.

9. DNA-Fragment nach einem der Ansprüche 1 bis 8, worin die Gene bioD und bioA derart angeordnet sind, daß der 3'-Terminus des bioD-Gens die Ribosomenbindungsstelle für das bioA-Gen umfaßt.

10. Plasmid, enthaltend ein DNA-Fragment nach einem der Ansprüche 1 bis 9.

11. Plasmid pBO30A-15/9, wie hinterlegt in E. coli XL1-Blue, E. coli BM4062 oder E. coli ED8767 unter den Hinterlegungsnummern DSM 7246, DSM 7247 bzw. DSM 8554.

12. Plasmid pBO47, wie hinterlegt in Agrobacterium/Rhizobium sp. HK4 unter der Hinterlegungsnummer DSM 8555.

13. Mikroorganismus, enthaltend ein DNA-Fragment oder ein Plasmid nach einem der Ansprüche 1 bis 12.

14. E. coli XL1-Blue, E. coli BM4062 und E. coli ED8767, jeweils enthaltend Plasmid pB030A-13/9, hinterlegt unter den Hinterlegungsnummern DSM 7246, DSM 7247 bzw. DSM 8554, sowie deren genetische Varianten und Mutanten.

15. Agrobacterium Rhizobium sp HK4, enthaltend Plasmid pB047, hinterlegt unter der Hinterlegungsnummer DSM 8555, sowie dessen genetische Varianten und Mutanten.

16. Biotechnologisches Verfahren zur Biotinsynthese, worin eine verstoffwechselbare Kohlenstoffquelle mittels eines Mikroorganismus nach einem der Ansprüche 13 bis 15 zu Biotin fermentiert wird.

## Claims

1. DNA fragment which comprises the genes for biotin biosynthesis bioB, bioF, bioC, bioD and bioA from enterobacteria or their functionally equivalent genetic variants and mutants, characterized in that the genes are arranged in a transcription unit and in one transcription direction and are transcribed from a common promoter.

2. DNA fragment according to Claim 1, wherein the enterobacteria are selected from the group of genera Escherichia, Salmonella and Citrobacter.

3. DNA fragment according to Claim 1 or 2, wherein the enterobacteria are selected from the species Escherichia coli.

4. DNA fragment according to any of Claims 1 to 3, wherein the common promoter is the tac promoter.

5. DNA fragment according to any of Claims 1 to 4, wherein the gene-regulatory element of the transcription unit directly linked to the bioB gene comprises the sequence:

6. DNA fragment according to any of Claims 1 to 4, wherein the gene-regulatory element of the transcription unit directly linked to the bioB gene comprises the sequence:

7. DNA fragment according to any of Claims 1 to 4, wherein the gene-regulatory element of the transcription unit directly linked to the bioB gene comprises the sequence:

8. DNA fragment according to any of Claims 1 to 7, wherein the distance between the bioD and bioA genes which are consecutive in the transcription unit is not more than 50 bp.

9. DNA fragment according to any of Claims 1 to 8, wherein the bioD and bioA genes are arranged in such a way that the 3' terminus of the bioD gene comprises the ribosome binding site for the bioA gene.

10. Plasmid containing a DNA fragment according to any of Claims 1 to 9.

11. Plasmid pBO30A-15/9 as deposited in E. coli XL1-Blue, E. coli BM4062 or E. coli ED8767 under deposit numbers DSM 7246, DSM 7247 or DSM 8554 respectively.

12. Plasmid pBO47 as deposited in Agrobacterium/Rhizobium sp. HK4 under deposit number DSM 8555.

13. Microorganism containing a DNA fragment or a plasmid according to any of Claims 1 to 12.

14. E. coli XL1-Blue, E. coli BM4062 and E. coli ED8767, each containing plasmid pB030A-13/9, deposited under deposit numbers DSM 7246, DSM 7247 and DSM 8554, respectively, and their genetic variants and mutants.

15. Agrobacterium/Rhizobium sp HK4, containing plasmid pB047, deposited under deposit number DSM 8555, and its genetic variants and mutants.

16. Biotechnological method for biotin synthesis, wherein a metabolizable carbon source is fermented to biotin by means of a microorganism according to any of Claims 13 to 15.

## Revendications

1. Fragment d'ADN qui comprend les gènes pour la biosynthèse de la biotine bioB, bioF, bloc, bioD et bioA d'entérobactéries ou leurs variants et mutants génétiques équivalents du point de vue fonctionnel, caractérisé en ce que les gènes sont disposés dans une unité de transcription et dans une direction de transcription et sont transcrits par un promoteur commun.

2. Fragment d'ADN selon la revendication 1 où les entérobactéries sont choisies dans le groupe des genres Escherichia, Salmonella et Citrobacter.

3. Fragment d'ADN selon la revendication 1 ou 2 où les entérobactéries sont choisies dans l'espèce Escherichia coli.

4. Fragment d'ADN selon l'une des revendications 1 à 3 où le promoteur commun est le promoteur tac.

5. Fragment d'ADN selon l'une des revendications 1 à 4 où l'élément régulateur de gènes de l'unité de transcription en liaison directe avec le gène bioB comprend la séquence:

6. Fragment d'ADN selon l'une des revendications 1 à 4 où l'élément régulateur de gènes de l'unité de transcription en liaison directe avec le gène bioB comprend la séquence:

7. Fragment d'ADN selon l'une des revendications 1 à 4 où l'élément régulateur de gènes de l'unité de transcription en liaison directe avec le gène bioB comprend la séquence:

8. Fragment d'ADN selon l'une des revendications 1 à 7 où la distance entre les gènes bioD et bioA qui se suivent dans l'unité de transcription n'est pas supérieure à 50 pb.

9. Fragment d'ADN selon l'une des revendications 1 à 8 où les gènes bioD et bioA sont agencés de telle manière que l'extrémité 3' du gène bioD comprend le site de fixation ribosomique pour le gène bioA.

10. Plasmide contenant un fragment d'ADN selon l'une des revendications 1 à 9.

11. Plasmide pBO30A-15/9, tel que déposé dans E. coli XL1-Blue, E. coli BM4062 ou E. coli ED8767 sous les numéros de dépôt DSM 7246, DSM 7247 et DSM 8554.

12. Plasmide pBO47, tel que déposé dans Agrobacterium/Rhizobium sp. HK4 sous le numéro de dépôt DSM 8555.

13. Micro-organisme contenant un fragment d'ADN ou un plasmide selon l'une des revendications 1 à 12.

14. E. coli XL1-Blue, E. coli BM4062 et E. coli ED8767 contenant chacun le plasmide pBO30A-13/9, déposés sous les numéros de dépôt DSM 7246, DSM 7247 et DSM 8554, ainsi que leurs variants et mutants génétiques.

15. Agrobacterium/Rhizobium sp HK4 contenant le plasmide pBO47, déposé sous le numéro de dépôt DSM 8555, ainsi que ses variants et mutants génétiques.

16. Procédé biotechnologique de biosynthèse où une source de carbone métabolisable est convertie en biotine par fermentation au moyen d'un micro-organisme selon l'une des revendications 13 à 15.
